# EUROPEAN PATENT APPLICATION

(11) **EP 4 528 274 A2**
(43) Date of publication of application: **26.03.2025**
(21) Application number: 24210986.6
(22) Date of filing: 01.04.2021
(51) Int. Cl.: G01N 33/50

(54) **BIOMARKERS FOR NEUROPATHOLOGICAL CONDITIONS AND METHODS FOR DIAGNOSIS AND MONITORING THE EFFICACY OF TREATMENT**

(30) Priority: 01.04.2020 GB 202004832
(62) Divisional of application: 21723440.0
(71) Applicant: Instituto de Medicina Molecular João Lobo Antunes, 1649-028 Lisboa (PT); Universidade de Coimbra, 3004-531 Coimbra (PT); Faculdade de Medicina da Universidade de Lisboa, 1649-028 Lisboa (PT)
(72) Inventor: DE OLIVEIRA DIÓGENES NOGUEIRA, Maria José, Lisboa (PT); FONSECA GOMES, João Filipe, Lisboa (PT); SANTOS, André Jerónimo, Lisboa (PT); FERREIRA DE SOUSA SEBASTIÃO, Ana Maria, Lisboa (PT); BANDEIRA DUARTE, Carlos, Coimbra (PT)
(74) Representative: Pereira da Cruz, Joao

(57) **Abstract**

The invention relates to the use of biomarkers of TrkB-FL, TrkB-ICD, TrkB-T', the TrkB-T':TrkB-FL ratio, or the TrkB-ICD:TrkB-FL ratio in the *in vitro* diagnosis of Alzheimer Disease (AD) or for determining the stage of AD, methods for their use, methods of diagnosis, methods of monitoring disease progression, in neuropathological diseases, in particular for Alzheimer's disease.

## Description

### FIELD OF THE INVENTION

The present invention relates to the identification of biomarkers for neuropathological conditions, in particular Alzheimer's disease (AD). Provided herein are biomarkers, methods for their use, methods of diagnosis, methods of monitoring disease progression, and methods for monitoring medicament efficacy, in particular for Alzheimer's disease.

### PRIOR ART

Brain-derived Neurotrophic Factor (BDNF) and its full-length cognate receptor, tropomyosin-receptor kinase B-full-length (TrkB-FL), are capable of regulating neuronal differentiation, growth and synaptic transmission and plasticity. This signalling pathway forms one of the most important neuroprotective systems in the developing and mature brain¹.

Dysregulation of BDNF function has been implicated in several diseases leading to sustained neurodegeneration, including Alzheimer's disease (AD). AD is already the most common form of dementia worldwide, and its prevalence is expected to increase further.

BDNF and TrkB-FL levels were shown to be decreased in the brain of human patients with AD2-4. The present inventors have previously described the mechanism through which amyloid-β (Aβ) leads to TrkB-FL cleavage. Briefly, the Aβ peptide recruits extra-synaptic NMDARs to upregulate intracellular Ca²⁺ levels⁵, leading to calpain overactivation and TrkB-FL cleavage⁶. This cleavage generates two products: (1) a membrane-bound truncated receptor (TrkB-T'), with no apparent function; and (2) an intracellular fragment (TrkB-ICD). The inventors show that this intracellular fragment accumulates in the cell nucleus overtime and displays a strong tyrosine kinase activity⁷.

There is a need to develop strategies for the diagnosis of neuropathological conditions, and in particular AD. The present solution intended to innovatively overcome such issues.

### GENERAL DESCRIPTION

In a preferred embodiment, the present disclosure refers to the use of TrkB-FL, TrkB-ICD, TrkB-T', the TrkB-T':TrkB-FL ratio, or the TrkB-ICD:TrkB-FL ratio in the *in vitro* diagnosis of Alzheimer Disease (AD) or for determining the stage of AD.

In a further embodiment, the present disclosure refers to a method for diagnosing AD or for determining the stage of AD, comprising:
i) comparing the amount of TrkB-FL and/or TrkB-ICD measured in a sample obtained from a subject to a reference amount, and;
ii) a) determining that said subject has AD, or determining that said subject has a more advanced stage of AD than that represented by the reference amount, if said amount of TrkB-FL is lower than said reference amount and/or if said amount of TrkB-ICD is higher than said reference amount; or
   b) determining that said subject has the same stage of AD or AD diagnosis as that represented by the reference amount if said amount of TrkB-FL and/or TrkB-ICD is the same or similar to said reference amount; or
   c) determining that said subject does not have AD, or determining that said subject has a less advanced stage of AD than that represented by the reference amount, if said amount of TrkB-FL is higher than said reference amount and/or if said amount of TrkB-ICD is lower than said reference amount;
said method optionally comprising prior to step i), measuring the amount of TrkB-FL and/or TrkB-ICD in a sample obtained from a subject.

In a further embodiment, the present disclosure refers to a method for diagnosing AD or for determining the stage of AD, comprising:
i) comparing the amount of TrkB-T' measured in a sample obtained from a subject to a reference amount, and
ii) a) determining that said subject has AD, or determining that said subject has a more advanced stage of AD than that represented by the reference amount, if said amount of TrkB-T' is higher than said reference amount; or b) determining that said subject has the same stage of AD or AD diagnosis as that represented by the reference amount if said amount of TrkB-T' is the same or similar to said reference amount; or c) determining that said subject does not have AD, or determining that said subject has a less advanced stage of AD than that represented by the reference amount, if said amount of TrkB-T' is lower than said reference amount;
said method optionally comprising prior to step i), measuring the amount of TrkB-T' in a sample obtained from a subject.

In a further embodiment, the present disclosure refers to a method, wherein the reference amount is representative of the amount of TrkB-T', TrkB-FL, and/or TrkB-ICD in a subject without AD, in a subject with mild AD, in a subject with moderate AD, in a subject with severe AD, determined from said subject before disease onset, determined from said subject after diagnosis of mild AD, determined from said subject after diagnosis with moderate AD, determined from said subject after diagnosis with severe AD, or determined from said subject before treatment.

In a further embodiment, the present disclosure refers to a method for diagnosing AD or for determining the stage of AD, comprising:
i) comparing the TrkB-ICD:TrkB-FL ratio measured in a sample obtained from a subject to a reference value, and
ii) a) determining that said subject has AD or determining that said subject has a more advanced stage of AD than that represented by the reference value, if said TrkB-ICD:TrkB-FL ratio is higher than said reference value; or b) determining that said subject has the same stage of AD or AD diagnosis as that represented by the reference value if said TrkB-ICD:TrkB-FL ratio is the same or similar to said reference value; or c) determining that said subject does not have AD, or determining that the subject has a less advanced stage of AD than that represented by the reference value, if said TrkB-ICD:TrkB-FL ratio is lower than said reference value;
wherein said method comprises prior to step i), measuring the amount of TrkB-FL and TrkB-ICD in a sample obtained from the subject, and calculating the TrkB-ICD:TrkB-FL ratio in said sample.

In a further embodiment, the present disclosure refers to a method for diagnosing AD or for determining the stage of AD, comprising:
i) comparing the TrkB-T':TrkB-FL ratio measured in a sample obtained from a subject to a reference value, and
ii) a) determining that said subject has AD or determining that said subject has a more advanced stage of AD than that represented by the reference value, if said TrkB-T':TrkB-FL ratio is higher than said reference value; or b) determining that said subject has the same stage of AD or AD diagnosis as that represented by the reference value if said TrkB-T':TrkB-FL ratio is the same or similar to said reference value; or c) determining that said subject does not have AD, or determining that the subject has a less advanced stage of AD than that represented by the reference value, if said TrkB-T':TrkB-FL ratio is lower than said reference value;
wherein said method comprises prior to step i), measuring the amount of TrkB-FL and TrkB-T' in a sample obtained from the subject, and calculating the TrkB-T':TrkB-FL ratio in said sample.

In a further embodiment, the present disclosure refers to a method, wherein the reference value is representative of the TrkB-ICD:TrkB-FL ratio or the TrkB-T':TrkB-FL ratio in a subject without AD, in a subject with mild AD, in a subject with moderate AD, in a subject with severe AD, determined from said subject before disease onset, determined from said subject after diagnosis of mild AD, determined from said subject after diagnosis with moderate AD, determined from said subject after diagnosis with severe AD, or determined from said subject before treatment.

In a further embodiment, the present disclosure refers to a method, wherein said subject is diagnosed with AD if the subject's measured TrkB-ICD:TrkB-FL ratio or TrkB-T':TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to a reference value representative of a subject without AD.

In a further embodiment, the present disclosure refers to a method for diagnosing AD, comprising:
i) comparing the TrkB-ICD:TrkB-FL ratio or the TrkB-T':TrkB-FL ratio measured in a sample obtained from a first subject with mild cognitive impairment to a reference value obtained from at least one subject without AD, and
ii) a) determining that the first subject has AD if said TrkB-ICD:TrkB-FL ratio or TrkB-T':TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to said reference value; or b) determining that the first subject does not have AD if said TrkB-ICD:TrkB-FL ratio or TrkB-T':TrkB-FL ratio is not increased compared to said reference value;
wherein said method optionally further comprises prior to step i): measuring the amount of TrkB-FL and TrkB-ICD in a sample obtained from the first subject with mild cognitive impairment, and calculating the TrkB-ICD:TrkB-FL ratio; and/or, measuring the amount of TrkB-FL and TrkB-T' in a sample obtained from the first subject with mild cognitive impairment, and calculating the TrkB-T':TrkB-FL ratio.

In a further embodiment, the present disclosure refers to a method for monitoring the efficacy of an AD treatment with an agent, comprising:
i) calculating an expected amount of TrkB-T', TrkB-FL, and/or TrkB-ICD based upon an amount of TrkB-T', TrkB-FL, and/or TrkB-ICD measured in a first sample obtained from a subject with AD and the time elapsed since the first sample was obtained; and;
ii) comparing: an amount of TrkB-T', TrkB-FL, and/orTrkB-ICD measured in a second sample obtained from the subject after administration of an agent to the subject, to said expected amount of TrkB-T', TrkB-FL, and/or TrkB-ICD; and
iii) a) determining that the treatment is efficacious if the amount of TrkB-FL in the second sample is higher than the expected amount ofTrkB-FL, if the amount ofTrkB-ICB in the second sample is lower than the expected amount ofTrkB-ICB, and/or if the amount of TrkB-T' in the second sample is lower than the expected amount of TrkB-T'; or b) determining that the treatment is not efficacious if the amount of TrkB-FL in the second sample is lower than or the same as the expected amount of TrkB-FL, if the amount ofTrkB-ICB in the second sample is higher than or the same as the expected amount of TrkB-ICB, and/or if the amount of TrkB-T' in the second sample is higher than or the same as the expected amount of TrkB-T';
wherein said method optionally further comprising, prior to step i), measuring the amount of TrkB-T', TrkB-FL, and/or TrkB-ICD in a first sample obtained from a subject, and prior to step ii), measuring the amount of TrkB-T', TrkB-FL, and TrkB-ICD in a second sample obtained from said subject after administration of an agent to the subject.

In a further embodiment, the present disclosure refers to a method for monitoring the efficacy of an AD treatment with an agent, comprising:
i) calculating an expected TrkB-ICD:TrkB-FL ratio orTrkB-T':TrkB-FL ratio based upon a TrkB-ICD:TrkB-FL ratio or TrkB-T':TrkB-FL ratio measured in a first sample obtained from a subject with AD and the time elapsed since the first sample was obtained; and
ii) comparing: a TrkB-ICD:TrkB-FL ratio or TrkB-T':TrkB-FL ratio measured in a second sample obtained from the subject after administration of an agent to the subject, to said expected TrkB-ICD:TrkB-FL ratio or TrkB-T':TrkB-FL ratio; and
iii) a) determining that the treatment is efficacious if the TrkB-ICD:TrkB-FL ratio in the second sample is lower than the expected TrkB-ICD:TrkB-FL ratio, or determining that the treatment is efficacious if the TrkB-T':TrkB-FL ratio in the second sample is lower than the expected TrkB-T':TrkB-FL ratio; or b) determining that the treatment is not efficacious if the TrkB-ICD:TrkB-FL ratio in the second sample is higher than or the same as the expected TrkB-ICD:TrkB-FL ratio, or determining that the treatment is not efficacious if the TrkB-T':TrkB-FL ratio in the second sample is higher than or the same as the expected TrkB-T':TrkB-FL ratio;
wherein said method further comprises prior to step i): measuring the amount of TrkB-FL and TrkB-ICD in the first sample obtained from the subject, and calculating the TrkB-ICD:TrkB-FL ratio in the first sample and, prior to step ii), measuring the amount of TrkB-FL and TrkB-ICD in the second sample obtained from the subject after administration of an agent to the subject, and calculating the TrkB-ICD:TrkB-FL ratio in the second sample; or measuring the amount of TrkB-FL and TrkB-T' in the first sample obtained from the subject, and calculating the TrkB-T':TrkB-FL ratio in the first sample and, prior to step ii), measuring the amount of TrkB-FL and TrkB-T' in the second sample obtained from the subject after administration of an agent to the subject, and calculating the TrkB-T':TrkB-FL ratio in the second sample.

In a further embodiment, the present disclosure refers to a method for monitoring the efficacy of an AD treatment with an agent, comprising:
i) measuring the amount of TrkB-FL and TrkB-ICD in a first sample obtained from a subject with AD, and
ii) calculating the TrkB-ICD:TrkB-FL ratio in said first sample, and
iii) administering an agent to said subject, and
iv) measuring the amount of TrkB-FL and TrkB-ICD in a second sample obtained from said subject, and
v) calculating the TrkB-ICD:TrkB-FL ratio in said second sample, and
vi) a) determining that the treatment is efficacious if the TrkB-ICD:TrkB-FL ratio in the second sample is lower than the TrkB-ICD:TrkB-FL ratio in the first sample; or b) determining that the treatment is not efficacious if the TrkB-ICD:TrkB-FL ratio in the second sample is higher than the TrkB-ICD:TrkB-FL ratio in the first sample.

In a further embodiment, the present disclosure refers to a method for monitoring the efficacy of an AD treatment with an agent, comprising:
i) measuring the amount of TrkB-FL and TrkB-T' in a first sample obtained from a subject with AD, and
ii) calculating the TrkB-T':TrkB-FL ratio in said first sample, and
iii) administering an agent to said subject, and
iv) measuring the amount of TrkB-FL and TrkB-T' in a second sample obtained from said subject, and
v) calculating the TrkB-T':TrkB-FL ratio in said second sample, and
vi) a) determining that the treatment is efficacious if the TrkB-T':TrkB-FL ratio in the second sample is lower than the TrkB-T':TrkB-FL ratio in the first sample; or b) determining that the treatment is not efficacious if the TrkB-T':TrkB-FL ratio in the second sample is higher than the TrkB-T':TrkB-FL ratio in the first sample.

In a further embodiment, the present disclosure refers to a method, wherein said sample is cerebrospinal fluid (CSF), blood, plasma, serum, or saliva.

Any of the aspects and embodiments described herein can be combined with any other aspect or embodiment as disclosed here in the Summary of the Invention, in the Drawings, and/or in the Detailed Description of the Invention, including the below specific, non-limiting, examples/embodiments of the present invention.

Unless otherwise defined, all technical and scientific terms used herein have the same meaning as commonly understood by one of ordinary skill in the art to which this application belongs.

Although methods and materials similar to or equivalent to those described herein can be used in the practice and testing of the application, suitable methods and materials are described below. All publications, patent applications, patents, and other references mentioned herein are incorporated by reference.

The references cited herein are not admitted to be prior art to the claimed application. In the case of conflict, the present specification, including definitions, will control. In addition, the materials, methods, and examples are illustrative only and not intended to be limiting.

Other features and advantages of the disclosure will become apparent from the following detailed description in conjunction with the examples.

### DESCRIPTION OF FIGURES

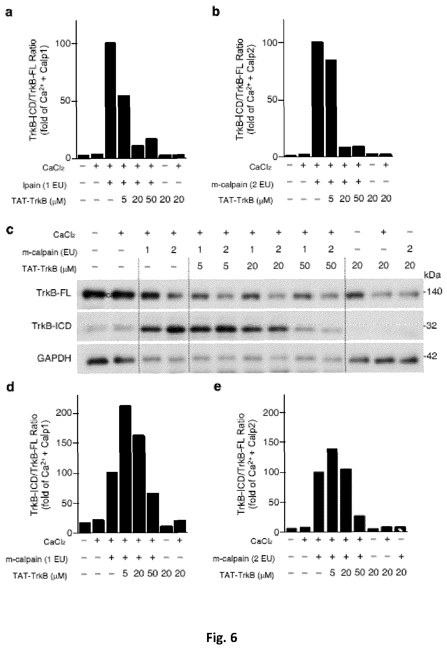

The following figures provide preferred embodiments for illustrating the disclosure and should not be seen as limiting the scope of invention.

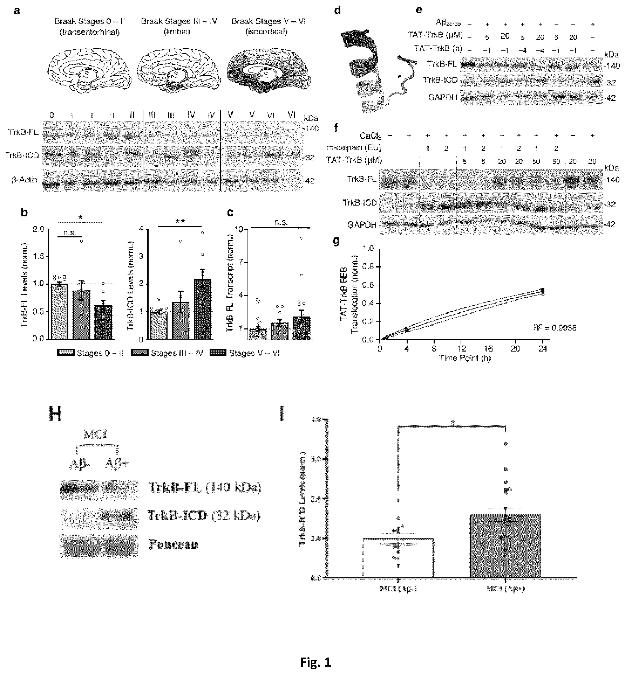

**Figure 1** shows that **TrkB-FL cleavage increases during AD progression and is prevented by TAT-TrkB.** The levels of cerebral TrkB-FL receptors decrease in later stages of the disease concomitantly to an increase of TrkB-ICD levels. In cerebrospinal fluid (CSF), patients with Mild Cognitive Impairment (MCI) due to Alzheimer's disease (MCI Aβ+) have significantly higher levels of TrkB-ICD when compared with non-AD patients (MCI Aβ-).
   a. Classification of AD pathology according to Braak staging (top). Western-blot of human samples probed with anti-Trk C-terminal and anti-β-actin antibodies (bottom).
   b. Analysis of bands intensities represented in a. Data normalized for Braak stages 0-II (p<0.05 and p<0.01, One-way ANOVA, followed by Bonferroni's post-test, n=7-11).
   c. Analysis of TrkB-FL transcript (NTRK2 gene) levels normalized to SV2B transcript. Data normalized for Braak stages 0-II (One-way ANOVA, followed by Bonferroni's post-test, n=11-27).
   d. Analysis of the TAT-TrkB predicted 3D structure; asterisk marks cleavage site.
   e. Western-blot of neuronal cultures. Aβ-driven cleavage of TrkB-FL was prevented by TAT-TrkB peptide (5 and 20 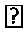 M) incubated 1h or 4h before Aβ incubation.
   f. Western-blot of adult rat brain cortex homogenate. TAT-TrkB (20, 50 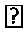 M) prevented calpain-mediated cleavage of TrkB-FL. Quantifications in Fig.6a-b.
   g. Brain endothelial barrier (BEB) in vitro translocation kinetics of TAT-TrkB.
      Complete statistical details in Supplementary Table 2.
   h. Representative western-blot of human CSF samples from one patient with Mild Cognitive Impairment (MCI) (MCI Aβ-) and one with MCI due to AD (MCI Aβ+), probed with anti-Trk C-terminal. Ponceau used as loading control.
   i. Average immunoreactive band intensity of TrkB-ICD in CSF from MCI (Aβ-) and from AD (MCI Aβ+) patients (values are normalized to MCI Aβ-). TrkB-ICD levels significantly increase in the CSF of MCI Aβ+ patients when compared to MCI Aβ- patients (*p<0.05, Mann-Whitney non-parametric test, n=12-20).

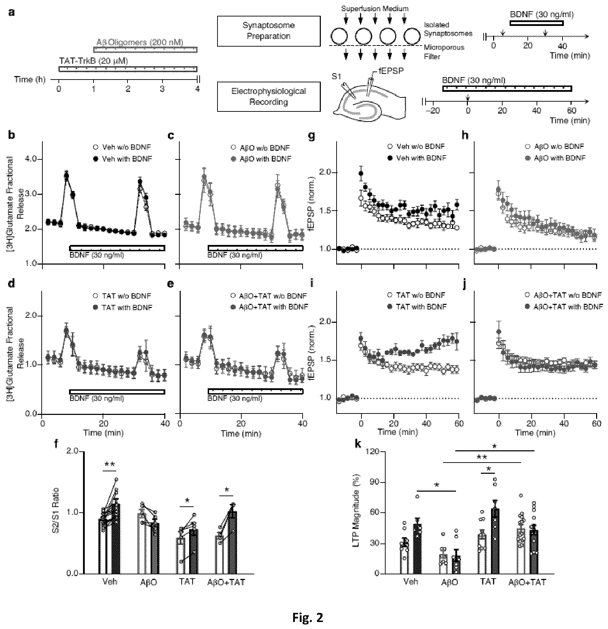

**Figure 2** shows that **TAT-TrkB prevented the loss of BDNF synaptic effects.**
   **a.** Experimental design.
   **b-e.** Time courses of fractional release of [³H]glutamate from synaptosomes, evoked by two 15 mM K⁺ stimulus (at 5-7 min and 29-31 min), in the absence or presence of BDNF (30 ng/ml) (**b**), Aβ (200 nM) oligomers (**c**), TAT-TrkB (20 µM) (**d**) and both TAT-TrkB and Aβ oligomers (**e**)**.**
   **f.** Histogram depicting the S2/S1 ratios from the experiments in **b-e.**
   **g-j.** Time courses of the field excitatory postsynaptic potential (fEPSP) slope after delivery (0 min) of a θ-burst in the absence or presence of BDNF (30 ng/ml) to hippocampal slices exposed to vehicle (**g**) or Aβ (200 nM) oligomers (**h**), TAT-TrkB (20 µM) (i) and both TAT-TrkB and Aβ oligomers (j).
   **k.** Histogram depicting LTP magnitudes from the recordings in **g-j.**
      Complete statistical details in Supplementary Table 2.

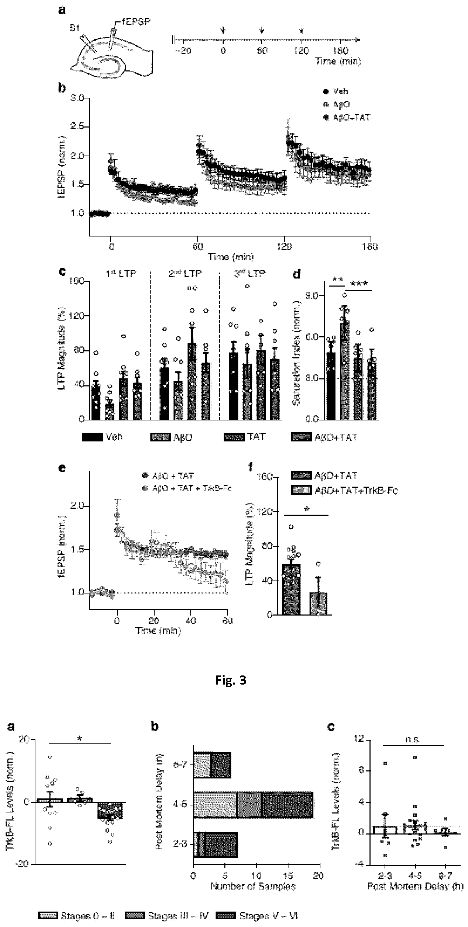

**Figure 3** is a representation of **TAT-TrkB prevented desaturation of hippocampal LTP.**
   **a.** Experimental design.
   **b.** Time course of fEPSP slope after three θ-burst, separated by 1h-intervals, to hippocampal slices exposed to vehicle, Aβ (200 nM) oligomers or both Aβ oligomers and TAT-TrkB (20 µM).
   **c.** Histogram depicting the magnitudes of the first, second and third LTPs from the recordings shown in **b.,** and from slices exposed to TAT-TrkB (20 µM) alone.
   **d.** Histogram depicting the Saturation Index (SI) pertaining to the recordings in **b.**
   **e.** Time courses of fEPSP slope after delivery (0 min) of a θ-burst to slices exposed to both Aβ oligomers and TAT-TrkB (20 µM), either superfused with BDNF's scavenger (TrkB-Fc, 2 µg/ml) or not (same data as in Fig.2j).
   **f.** Histogram depicting LTP magnitudes from the recordings in **e.**
      Complete statistical details in Supplementary Table 2.
**Figure 4** shows **Decreased TrkB-FL protein levels (detected by MS) are not affected by *post-mortem* delay in sample collection.**
   a. Proteomic analysis of TrkB-FL protein normalized to SV2B protein, using liquid-chromatography tandem mass spectrometry (LC-MS/MS). Samples obtained from human temporal cortical samples, with varying degree of AD-related pathology [n=11 (0-II), n=5 (III-IV) and n=16 (V-VI), mean±s.e.m., one-way ANOVA followed by a Bonferroni's multiple comparison test].
   b. Distribution of post-mortem delay (PMD) in time categories for the LC-MS/MS samples, according to Braak staging.
   c. TrkB-FL protein levels quantified in human temporal cortical samples using LC-MS/MS are plotted as a function of PMD time categories. TrkB-FL levels did not change significantly according to PMD in our cohort; thus, PMD is not a confounder of the association found between TrkB-FL levels and Braak staging [n=7 (2-3h), n=19 (4-5h) and n=6 (6-7h), mean±s.e.m., one-way ANOVA followed by a Bonferroni's multiple comparison test].
      Complete statistical details in Supplementary Table 2.

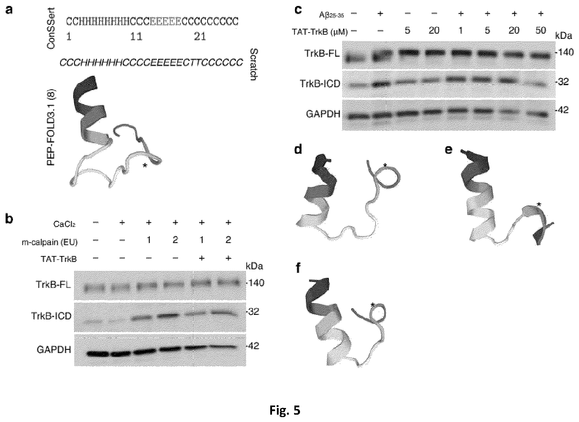

**Figure 5** represents **Failed and untested versions of the TAT-TrkB peptide.**
   a. Predicted secondary structure of TAT-TrkB without a linker sequence was obtained using various structure modelling softwares (ConSSert, Scratch and PEP-FOLD). ConSSert tool: C - coil; E - strand; H - helix. Scratch: C - linear structure; E - extended strand; H - α-helix; T - turn.
   b. Western-blot of adult rat brain cortex homogenate, showing decrease of TrkB-FL protein levels with concomitant TrkB-ICD formation when both exogenous m-calpain (1 or 2 enzymatic units, EU) and CaCl2 (2 mM) were added. TrkB-FL cleavage and TrkB-ICD formation were not prevented by the version of TAT-TrkB peptide (20 µM) in a. β-actin was used as loading control.
   c. Western-blot of DIV8 neuronal cultures showing TrkB-FL cleavage and TrkB-ICD formation with Aβ incubation, not prevented by the version of TAT-TrkB peptide (20 µM) in a (1, 5, 20 and 50 µM). β-actin was used as loading control.
   d-f. Analysis of the 3D structure, predicted using PEP-FOLD software, of the TAT-TrkB peptide using three different linkers sequences connecting the TAT sequence and the sequence spanning the cleavage site. This online server indicates that these three peptides have predicted stronger α-helix content than the TAT-TrkB version in a. After this 3D analysis, we synthesized the peptide with KK as linker (Fig. 1d), since the cleavage site is more exposed without compromising α-helix structure. Other linkers tested: PP, 2 Prolines (d), Q, 1 Glutamine (e) and E, 1 Glutamate (f).
**Figure 6** shows that **TAT-TrkB peptide** is **able to prevent TrkB-FL cleavage in a calpain *in vitro* assay from neuronal cultures lysate.**
   a-b. Analysis of bands intensities represented in Fig. 1f from densitometry quantification of TrkB-FL/TrkB-ICD ratio immunoreactivity, when assay was performed with m-calpain activity (EU, enzymatic units) of 1 EU (Fig. 6a) or 2 EU (Fig. 6b). Data is normalized for co-incubation of m-calpain (1/2 EU) and CaCl₂.
   c. Western-blot of DIV8 neuronal cultures lysate, showing decrease of TrkB-FL protein levels with concomitant TrkB-ICD formation when both exogenous m-calpain (1 or 2 EU) and CaCl₂ (2 mM) were added. TrkB-FL cleavage and TrkB-ICD formation were prevented in the presence of 50 µM TAT-TrkB peptide. GAPDH was used as loading control.
   **d-e.** Analysis of bands intensities represented in c. from densitometry quantification of TrkB-FL/TrkB-ICD ratio immunoreactivity, when assay was performed with m-calpain activity of 1 EU (Fig. 6d) or 2 EU (Fig. 6e). Data is normalized for co-incubation of m-calpain (1/2 EU) and CaCl₂.

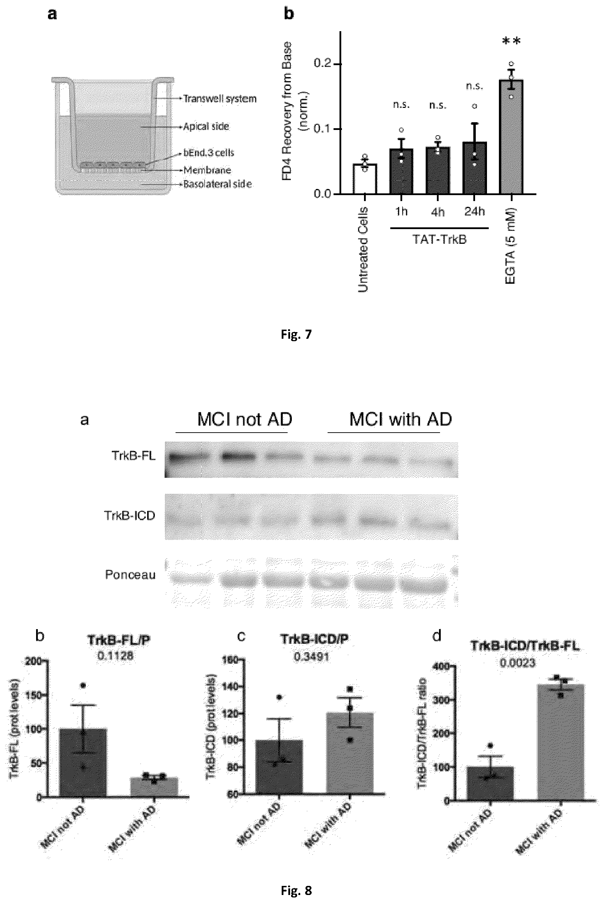

**Figure 7** shows that **the integrity of the Blood Endothelial Barrier (BEB) *in vitro* model** is **maintained after incubation with the TAT-TrkB peptide.**
   **a.** The BEB *in vitro* model comprises a transwell system with an insert in which bEnd.3 cells are cultured. In this model, the apical chamber replicates the blood compartment, while the basolateral chamber replicates the brain compartment. TAT-TrkB-6-FAM and FD4 probes were added to the apical side; samples were collected from both chambers and fluorescence was measured.
   **b.** After each TAT-TrkB peptide translocation assay (Fig. 1g), the FD4 fluorescent probe was added to the apical chamber and samples were collected 2 h thereafter. Untreated cells (negative control) and EGTA-treated cells (positive control) were also tested in parallel. The fluorescence detected in the basolateral chamber, normalized to the total fluorescence detected in both chambers, is expressed in the y axis. As expected, compared to untreated cells, EGTA significantly increased the permeability of the membrane, probably due to tight junction disruption. On the other hand, the clearance of FD4 from the apical chamber in the presence of TAT-TrkB peptide was negligible, arguing for the lack of fenestrations in the cell barrier and absence of paracellular leakage (n=3 for each condition, datapoints are averages of triplicates, mean±s.e.m., one-way ANOVA followed by a Bonferroni's multiple comparison test).
      For complete statistical details please consult Supplementary Table 2 of parent application EP4125999, which is herein incorporated by reference.
**Figure 8** is **Preliminary data regarding the possible biomarker application of TrkB-FL and TrkB-ICD protein levels, as well as TrkB-ICD/TrkB-FL ratio.**
   **a.** Western-blot of CSF samples obtained from patients diagnosed with and without AD pathophysiology probed with anti-Trk (C-14) antibody and staining for Ponceau as loading control.
   **b.** Quantification of TrkB-FL protein levels represented in a. and normalized to MCI patients without AD diagnosis.
   **c.** Quantification of TrkB-ICD protein levels represented in a. and normalized to MCI patients without AD diagnosis.
   **d.** Quantification of TrkB-ICD/TrkB-FL protein levels represented in a. and normalized to MCI patients without AD diagnosis.

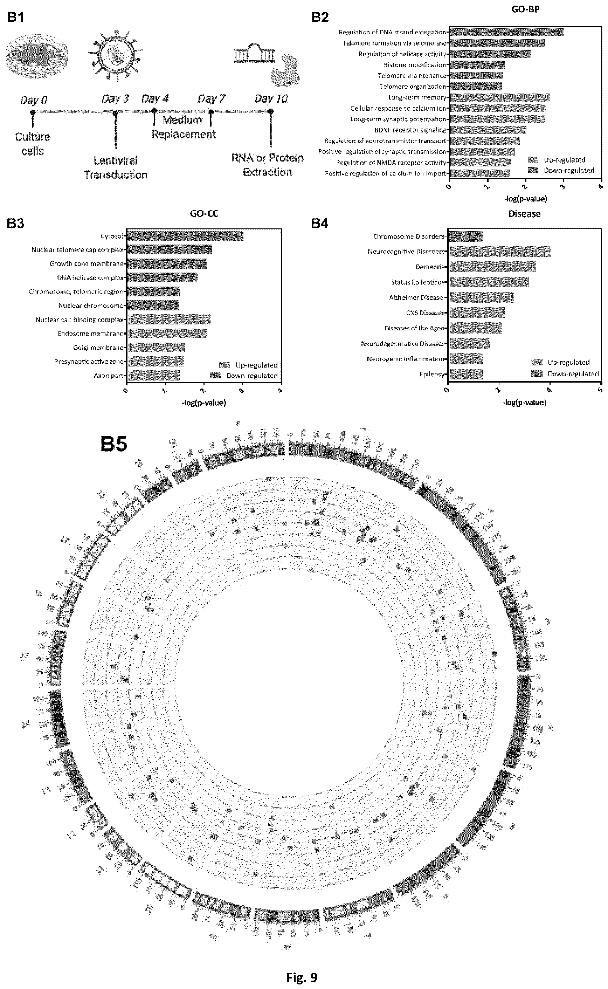

**Figure 9** is **TrkB-ICD overexpression in primary cortical neurons affects transcriptomics and is associated with Long-term memory, as well as Alzheimer's disease.**
   **B1.** Experimental timeline of lentiviral transduction of primary neurons and consequent RNA and protein extraction.
   **B2.** Identification of biological processes associated with up- and down-regulated genes, when TrkB-ICD-expressing neurons are compared to eGFP-expressing neurons.
   **B3.** Identification of cellular compartments associated with up- and down-regulated genes, when TrkB-ICD-expressing neurons are compared to eGFP-expressing neurons.
   **B4.** Identification of diseases associated with up- and down-regulated genes, when TrkB-ICD-expressing neurons are compared to eGFP-expressing neurons.
   **B5.** Ideogram - chromosome map - representing transcriptomic alterations induced by TrkB-ICD fragment.

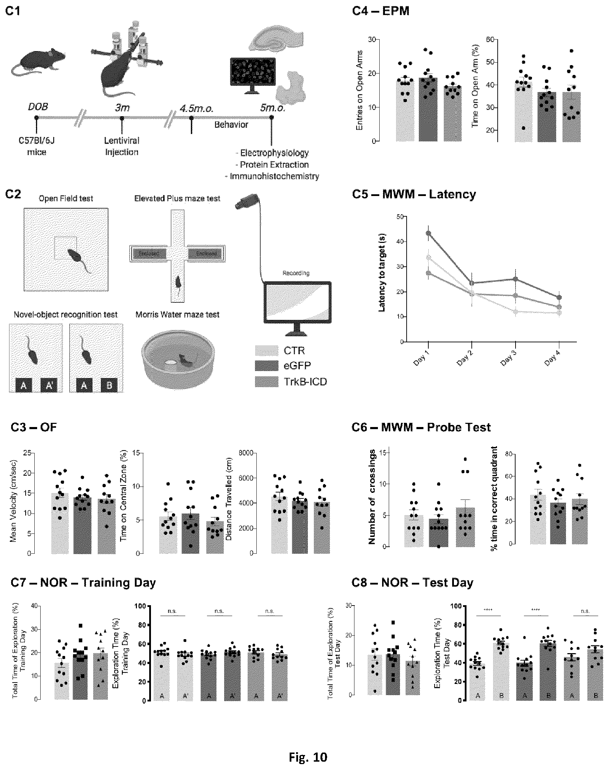

**Figure 10** is **TrkB-ICD overexpression in hippocampus of wild-type animals promotes memory impairment (in Novel-object recognition test), without locomotion and anxiety alterations.**
   **C1.** Experimental timeline of intra-hippocampal lentiviral transduction of wild-type animals and consequent behavioural,
   **C2.** Behavioural tests performed,
   **C3.** Open filed test: quantification of mean velocity and distance travelled (locomotion evaluators) and time spent on central zone (anxiety evaluator).
   **C4.** Elevated-plus maze test: quantification of entries and time spent on open arms (anxiety evaluator).
   **C5.** Morris water maze test: learning curve representing the latency to find the platform for 4 consecutive days.
   **C6.** Morris water maze test: quantification of number of platform crossings, as well as percentage of the time spent in the quadrant of the platform.
   **C7.** Novel-object recognition test: exploration time, total and per object, of training day. A and A' represent equal objects.
   **C8.** Novel-object recognition test: exploration time, total and per object, of training day. A and B represent different objects, being B the novel object.

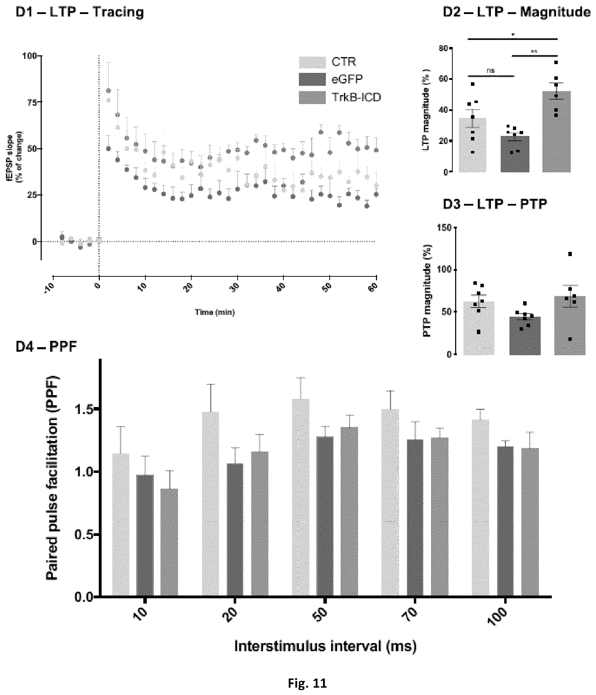

**Figure 11** is **TrkB-ICD overexpression in hippocampus of wild-type animals promote an increase of synaptic plasticity, without alterations in paired-pulse facilitation and post-tethanic pulses.**
   **D1.** Time courses of averaged normalized changes in fEPSP slope after delivery (0 min) of a weak θ-burst to hippocampal slices from control animals or animals-expressing TrkB-ICD or GFP. Data are mean±s.e.m.
   **D2.** Histogram depicting LTP magnitudes from the recordings in **D1.**
   **D3.** Histogram depicting PTP magnitudes from the recordings in **D1.**
   **D4.** Histograms of PPF at interstimulus of 10, 20, 50, 70, and 100 ms. The ordinates refer to an average value of PPF measured as a ratio between the second and the first fEPSP slope responses in hippocampal slices from control animals or animals-expressing TrkB-ICD or GFP. Data are mean±s.e.m.

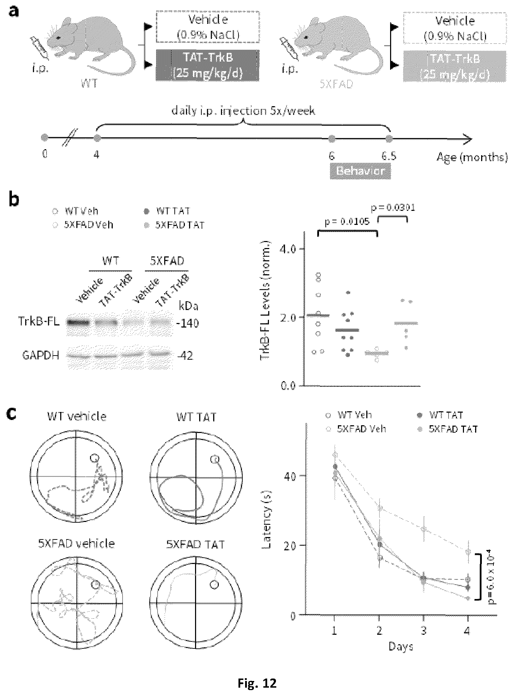

**Figure 12** is a *In vivo* pilot study to evaluate TAT-TrkB efficacy.
   **A.** Timeline of the experimental design.
   **B.** Quantification of TrkB-FL levels in both the hippocampus of wild type (WT, blue - two groups on the left of the graph) and SxFAD (yellow - two groups on the right of the graph) mice treated with either saline solution (Veh) or with TAT-TrkB (TAT)
   **C.** Comparison of the performance by WT and SxFAD treated with saline solution (veh) or with TAT-TrkB (TAT) in the spatial learning protocol of the Morris water maze. Learning curve of the mean escape latencies over the 4 days of acquisition training for WT and SxFAD animals untreated or treated with TAT-TrkB. The uppermost dotted line on the graph is SxFAD veh.

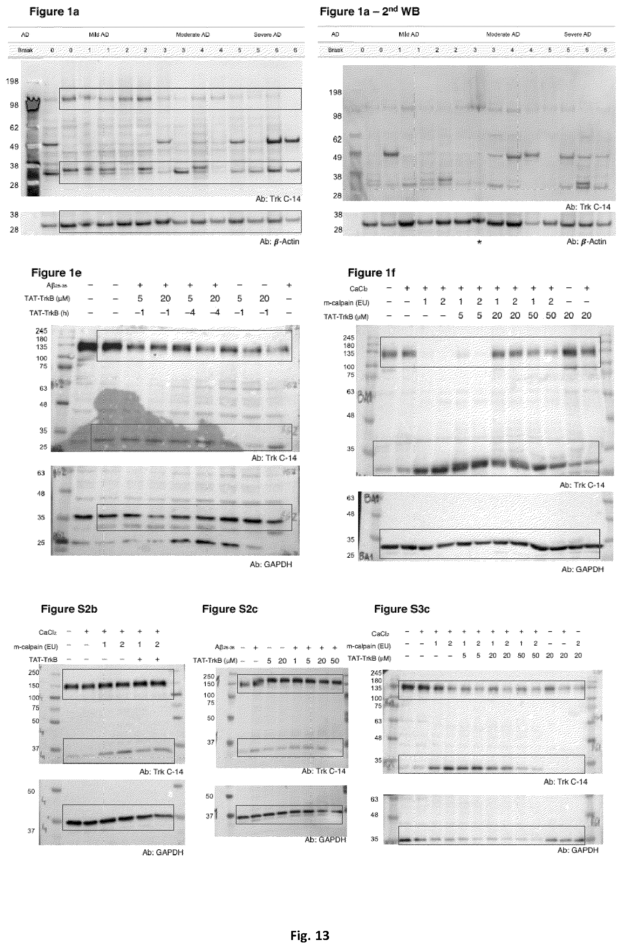

**Figure 13****. Images of uncropped gels and western blots with molecular weight standards.** Uncropped images of representative western blot images (squared in red) from Figures 1a, 1e and 1f and Figures 5b, 5c and 5c. Three different molecular weight standards were used: Invitrogen SeeBlue Plus2 Pre-stained Standard (Figure 1a), NZYTech Protein Marker II (Figures 1e, 1f and 6c) and Bio-Rad Precision Plus Protein (Figures 5b and 5c). In some cases, membranes were cut before antibody staining in order to allow two different stainings at the same time without reprobing. Sample identified with "*" in the "Figure 1a - 2nd WB" was not quantified, due to its low quality, possibly justified by compromised sample preparation.

### DETAILED DESCRIPTION

As previously shown by the inventors, the accumulation of amyloid-β during AD induces calpain activation which leads to TrkB-FL cleavage and impairment of BDNF neuromodulatory actions.

The inventors demonstrate in parent application EP4125999 (EP'999) that contacting a pharmaceutical composition according to the first object of patent EP'999 to cells, such as neuronal cells, can prevent amyloid-β-induced cleavage of TrkB-FL. Without being bound to a particular theory, the inventors hypothesise that the TrkB-FL portion is able to bind to the TrkB-FL cleavage site and hence protect TrkB-FL from calpain cleavage. Further, the inventors demonstrate that administration of said pharmaceutical composition to a subject in need of treatment will prevent the impairment of BDNF activity and prevent the accumulation of TrkB-ICD. Hence, the cognitive defects associated with AD will be treated, prevented, ameliorated, delayed, or reduced.

Prior to the present invention, it was not known that portions of TrkB-FL have the aforementioned property. Tejeda et al. teaches that a portion of TrkB-FL that spans the calpain cleavage site of TrkB-FL is not suitable for use as a neuroprotective agent⁸. The present inventors have overcome this technical prejudice. The inventors demonstrate that said TrkB-FL portion does have the desired activity, but that this activity can be lost if the portion of TrkB-FL is allowed to adopt particular conformations. As such, functional pharmaceutical compositions could be generated by ensuring that the portion of TrkB-FL is exposed outside of conformational structures, for instance outside of alpha-helical conformations.

The TrkB-FL may be human TrkB-FL. The amino acid sequence of TrkB-FL may be: 
MSPWLKWHGPAMARLWGLCLLVLGFWRASLACPTSCKCSSARIWCTEPSPGIVAFPRLEP
NSVDPENITEILIANQKRLEIINEDDVEAYVGLRNLTIVDSGLKFVAYKAFLKNSNLRHI
NFTRNKLTSLSRRHFRHLDLSDLILTGNPFTCSCDIMWLKTLQETKSSPDTQDLYCLNES
SKNMPLANLQIPNCGLPSARLAAPNLTVEEGKSVTLSCSVGGDPLPTLYWDVGNLVSKHM
NETSHTQGSLRITNISSDDSGKQISCVAENLVGEDQDSVNLTVHFAPTITFLESPTSDHH
WCIPFTVRGNPKPALQWFYNGAILNESKYICTKIHVTNHTEYHGCLQLDNPTHMNNGDYT
LMAKNEYGKDERQISAHFMGRPGVDYETNPNYPEVLYEDWTTPTDIGDTTNKSNEIPSTD
VADQSNREHLSVYAVVVIASVVGFCLLVMLLLLKLARHSKFGMKGPASVISNDDDSASPL
HHISNGSNTPSSSEGGPDAVIIGMTKIPVIENPQYFGITNSQLKPDTFVQHIKRHNIVLK
RELGEGAFGKVFLAECYNLCPEQDKILVAVKTLKDASDNARKDFHREAELLTNLQHEHIV
KFYGVCVEGDPLIMVFEYMKHGDLNKFLRAHGPDAVLMAEGNPPTELTQSQMLHIAQQIA
AGMVYLASQHFVHRDLATRNCLVGENLLVKIGDFGMSRDVYSTDYYRVGGHTMLPIRWMP
PESIMYRKFTTESDVWSLGVVLWEIFTYGKQPWYQLSNNEVIECITQGRVLQRPRTCPQE
VYELMLGCWQREPHTRKNIKSIHTLLQNLAKASPVYLDILG (SEQ ID NO: 1)
 The calpain cleavage site is between Asn⁵²⁰ and Ser⁵²¹ of SEQ ID NO: 1.

The amino acid sequence ofTrkB-ICD may be:

SQLKPDTFVQHIKRHNIVLKRELGEGAFGKVFLAECYNLCPEQDKILVAVKTLKDASDNA
RKDFHREAELLTNLQHEHIVKFYGVCVEGDPLIMVFEYMKHGDLNKFLRAHGPDAVLMAE
GNPPTELTQSQMLHIAQQIAAGMVYLASQHFVHRDLATRNCLVGENLLVKIGDFGMSRDV
YSTDYYRVGGHTMLPIRWMPPESIMYRKFTTESDVWSLGVVLWEIFTYGKQPWYQLSNNE
VIECITQGRVLQRPRTCPQEVYELMLGCWQREPHTRKNIKSIHTLLQNLAKASPVYLDIL
G (SEQ ID NO: 2)

The amino acid sequence ofTrkB-T' may be:

MSPWLKWHGPAMARLWGLCLLVLGFWRASLACPTSCKCSSARIWCTEPSPGIVAFPRLEP
NSVDPENITEILIANQKRLEIINEDDVEAYVGLRNLTIVDSGLKFVAYKAFLKNSNLRHI
NFTRNKLTSLSRRHFRHLDLSDLILTGNPFTCSCDIMWLKTLQETKSSPDTQDLYCLNES
SKNMPLANLQIPNCGLPSARLAAPNLTVEEGKSVTLSCSVGGDPLPTLYWDVGNLVSKHM
NETSHTQGSLRITNISSDDSGKQISCVAENLVGEDQDSVNLTVHFAPTITFLESPTSDHH
WCIPFTVRGNPKPALQWFYNGAILNESKYICTKIHVTNHTEYHGCLQLDNPTHMNNGDYT
LMAKNEYGKDERQISAHFMGRPGVDYETNPNYPEVLYEDWTTPTDIGDTTNKSNEIPSTD
VADQSNREHLSVYAVVVIASVVGFCLLVMLLLLKLARHSKFGMKGPASVISNDDDSASPL
HHISNGSNTPSSSEGGPDAVIIGMTKIPVIENPQYFGITN (SEQ ID NO: 3)

A "portion" of a protein, as used herein, is a peptide or polypeptide with a sequence that matches part, but not all, of the parent protein. The portion of a protein may be considered to be a fragment of said protein. In the embodiments, the portion of TrkB-FL is isolated from the full-length TrkB-FL protein but may be within a larger polypeptide or composition. For instance, the TrkB-FL portion may be included within a larger polypeptide that also includes polypeptide sequences not derived from TrkB-FL. The portion of TrkB-FL may be adjoined to amino acid sequences with which it is not associated in nature.

In an embodiment, a peptide which "spans" a specified site may comprise at least one amino acid residue to the N-terminal side of the specified site and at least one amino acid residue to the C-terminal side of the specified site. For example, to span the calpain cleavage site of TrkB-FL, the peptide may comprise at least a residue corresponding to Asn520 and at least a residue corresponding to Ser521. In an embodiment, the portion of TrkB-FL that spans the calpain cleavage site may include adequate residues on either side of the cleavage site such that the portion of TrkB-FL is able to prevent of the cleavage of TrkB-FL to produce TrkB-ICD. To confirm this activity, the portion may be tested using the in vitro experiments described herein.

The portion of TrkB-FL may include further modifications to the peptide, including naturally occurring modifications, any known post-translational modifications, glycosylation, pegylation, artificial modifications, or a combination.

In an embodiment, the portion of TrkB-FL may comprise 1 to 100, 1 to 75, 1 to 50, 1 to 40, 1 to 30, 1 to 25, 1 to 20, 1 to 15, or 1 to 10 of the residues on the N-terminal side of the TrkB-FL calpain cleavage site. For instance, in an embodiment, the portion of TrkB-FL may include at least 1 and no more than 10 of the consecutive residues that are N-terminal to the calpain cleavage site of TrkB-FL. In particular examples, the portion of TrkB-FL may include the sequence: N, TN, ITN, GITN (SEQ ID NO: 4), FGITN (SEQ ID NO: 5), YFGITN (SEQ ID NO: 6), QYFGITN (SEQ ID NO: 7), PQYFGITN (SEQ ID NO: 8), NPQYFGITN (SEQ ID NO: 9), or ENPQYFGITN (SEQ ID NO: 10). In a particular embodiment, the portion may comprise the five residues that are N-terminal to the TrkB-FL calpain cleavage site, for example FGITN (SEQ ID NO: 5). In an embodiment, the portion may comprise 1 to 100, 1 to 75, 1 to 50, 1 to 40, 1 to 30, 1 to 25, 1 to 20, 1 to 15, or 1 to 10 of the residues on the C-terminal side of the TrkB-FL calpain cleavage site. For instance, in an embodiment, the portion of TrkB-FL may include at least 1 and no more than 10 of the consecutive residues that are C-terminal to the calpain cleavage site of TrkB-FL. In particular examples, the portion of TrkB-FL may include the sequence: S, SQ, SQL, SQLK (SEQ ID NO: 11), SQLKP (SEQ ID NO: 12), SQLKPD (SEQ ID NO: 13), SQLKPDT (SEQ ID NO: 14), SQLKPDTF (SEQ ID NO: 15), SQLKPDTFV (SEQ ID NO: 16), or SQLKPDTFVQ (SEQ ID NO: 17). In a particular embodiment, the portion may comprise the five residues that are C-terminal to the TrkB-FL calpain cleavage site, for example SQLKP (SEQ ID NO: 12). In some embodiments, the aforementioned sequences on the N-terminal side and on the C-terminal side of the calpain cleavage site may be combined so that the TrkB-FL portion is 5 to 20 residues in length, 6 to 15 residues in length, 7 to 13 residues in length, 8 to 12 residues in length, 9 to 11 residues in length, or in particular embodiments 10 residues in length.

In another embodiment, the portion of TrkB-FL spans the calpain cleavage site and is at least 5, 6, 7, 8, 9, 10, 11, 12, 13, 14, 15, 16, 17, 18, 19, 20, 25, 30, 40, 50, 75, or 100 residues in length. In another embodiment, the portion of TrkB-FL spans the calpain cleavage site and is no more than 5, 6, 7, 8, 9, 10, 11, 12, 13, 14, 15, 16, 17, 18, 19, 20, 25, 30, 40, 50, 75, or 100 residues in length. In some embodiments, the portion of TrkB-FL spans the calpain cleavage site and is 5 to 20 residues in length, 6 to 15 residues in length, 7 to 13 residues in length, 8 to 12 residues in length, or 9 to 11 residues in length. In particular embodiments, the portion of TrkB-FL spans the calpain cleavage site and is 10 residues in length.

In a particular embodiment, the portion of TrkB-FL is according to the amino acid sequence FGITNSQLKP (SEQ ID NO: 18).

In another embodiment, the portion of TrkB-FL is according to the amino acid sequence PQYFGITNSQLKPDTF (SEQ ID NO: 19).

In an embodiment, the portion of TrkB-FL may be according to the amino acid sequence ITNSQLKPDTFVQH (SEQ ID NO: 20). In some embodiments, the portion of TrkB-FL may be as disclosed herein, and wherein the amino acid sequence is not according to SEQ ID NO: 20. In some embodiments, the portion of TrkB-FL spans the calpain cleavage site and is at least 5, 6, 7, 8, 9, 10, 11, 12, 13, or 14 residues in length, and is not according to SEQ ID NO: 20. In another embodiment, the portion ofTrkB-FL spans the calpain cleavage site and is no more than 14, 15, 16, 17, 18, 19, 20, 25, 30, 40, 50, 75, or 100 residues in length, and is not according to SEQ ID NO: 20.

In some embodiments, the portion of TrkB-FL may comprise an amino acid sequence according to SEQ ID NO: 18, SEQ ID NO: 19, or SEQ ID NO: 20, wherein the peptide comprises one or more substitutions, modifications, deletions, or replacements within this sequence. For example, one, two, three, four, or five substitutions of a residue within SEQ ID NO: 18, SEQ ID NO: 19, or SEQ ID NO: 20 for an alternative amino acid residue. In some embodiments, the polypeptide may comprise one to three substitutions. In particular embodiments, the one or more substitutions are conservative substitutions.

The inventors have previously shown that Aβ-induced calpain activation leads to TrkB cleavage and impairment of BDNF neuromodulatory actions⁶.

Herein, the inventors demonstrate that TrkB-FL levels decrease during the progression from mild, moderate, to severe Alzheimer's disease, whereas the levels of TrkB-ICD increase during this same progression. Hence, the levels of TrkB-FL or TrkB-ICD may be used as a biomarker indicating AD or stage of AD.

The inventors provide data herein validating the use of TrkB-T', TrkB-FL orTrkB-ICD quantification as a biomarker. Samples from patients with mild cognitive impairment not associated with AD were compared with samples from patients with mild cognitive impairment associated with AD. The inventors show that TrkB-T', TrkB-FL, TrkB-ICD, the TrkB-T':TrkB-FL ratio, or the TrkB-ICD:TrkB-FL ratio are all suitable for use as a biomarker for Alzheimer's disease. In particular, measurement of the TrkB-ICD:TrkB-FL ratio provided a robust indication of AD in subjects with mild cognitive impairment.

Notably, the inventors also examined the level of calpain activation during the progression of Alzheimer's disease. Calpain activation was found to be less useful as a biomarker in comparison to the level of TrkB-FL, the level of TrkB-ICD, or the TrkB-ICD:TrkB-FL ratio.

Therefore, in another aspect of the invention, there is provided the use of TrkB-FL, TrkB-ICD, TrkB-T', the TrkB-T':TrkB-FL ratio, or the TrkB-ICD:TrkB-FL ratio as a biomarker for Alzheimer's disease.

In an embodiment, there is disclosed a method for diagnosing AD or for determining the stage of AD, comprising: i) measuring the amount of TrkB-FL and/or TrkB-ICD in a sample obtained from a subject, and ii) comparing the amount of TrkB-FL and/or TrkB-ICD to a reference amount.

In an embodiment, there is disclosed a method for diagnosing AD or for determining the stage of AD, comprising comparing the amount of TrkB-FL and/or TrkB-ICD measured in a sample obtained from a subject to a reference amount.

A lower amount of TrkB-FL in the sample obtained from the subject in comparison to the reference amount may be indicative of AD or AD at a more advanced stage than that represented by the reference amount. The same or a similar amount of TrkB-FL may be indicative of AD at the same stage as that represented by the reference amount or of no AD if the reference represents a patient without AD. A higher amount of TrkB-FL in comparison to a reference amount may be indicative of no AD or AD at a less advanced stage than that represented by the reference amount. A higher amount of TrkB-ICD in the sample obtained from the subject in comparison to a reference amount may be indicative of AD or AD at a more advanced stage than that represented by the reference amount. The same or a similar amount of TrkB-ICD may be indicative of AD at the same stage as that represented by the reference amount or of no AD if the reference represents a patient without AD. A lower amount of TrkB-ICD in comparison to a reference amount may be indicative of no AD or AD at a less advanced stage than that represented by the reference amount. Hence, the method allows the determination of whether a subject has AD and/or of the stage of AD.

The reference amount may be representative of the amount of TrkB-FL and/or TrkB-ICD in a subject without AD. The reference amount may be representative of the amount of TrkB-FL and/or TrkB-ICD in a subject with mild AD. The reference amount may be representative of the amount of TrkB-FL and/or TrkB-ICD in a subject with moderate AD. The reference amount may be representative of the amount of TrkB-FL and/or TrkB-ICD in a subject with severe AD. The reference amount may have been determined from the same subject before disease onset, after diagnosis of AD, after diagnosis of mild AD, after diagnosis with moderate AD, or after diagnosis with severe AD. The reference amount may be obtained from a one or more, or a plurality of subjects. The reference amount may have been calculated from pre-collected data or from pre-collected samples. In some embodiments, the reference amount may be adjusted to take into account the age, sex, weight, ethnicity, amount of physical exercise, or other relevant factor of the subject to be tested. The reference amount may represent or take into account the natural variations of the biomarkers found in the representative population, and hence the reference value may be a range.

In an embodiment, the reference amount may be representative of mild AD, wherein the measured amount of TrkB-FL and/or TrkB-ICD in a sample obtained from a subject is indicative of the subject having AD at a more advanced stage if the amount of TrkB-FL is lower than the reference amount and/or the amount of TrkB-ICD is higher than the reference amount. In an embodiment, the reference amount may be representative of mild AD, wherein the measured amount of TrkB-FL and/or TrkB-ICD in a sample obtained from a subject is indicative of the subject having mild AD if the amount of TrkB-FL and/or TrkB-ICD is the same or similar to the reference amount. In an embodiment, the reference amount may be representative of mild AD, wherein the measured amount of TrkB-FL and/orTrkB-ICD in a sample obtained from a subject is indicative of the subject not having AD or having AD at a less advanced stage if the amount of TrkB-FL is higher than the reference amount and/or the amount of TrkB-ICD is lower than the reference amount.

In an embodiment, the reference amount may be representative of moderate AD, wherein the measured amount of TrkB-FL and/or TrkB-ICD in a sample obtained from a subject is indicative of the subject having AD at a more advanced stage if the amount of TrkB-FL is lower than the reference amount and/or the amount of TrkB-ICD is higher than the reference amount. In an embodiment, the reference amount may be representative of moderate AD, wherein the measured amount of TrkB-FL and/or TrkB-ICD in a sample obtained from a subject is indicative of the subject having moderate AD if the amount of TrkB-FL and/or TrkB-ICD is the same or similar to the reference amount. In an embodiment, the reference amount may be representative of moderate AD, wherein the measured amount of TrkB-FL and/or TrkB-ICD in a sample obtained from a subject is indicative of the subject not having AD or having AD at a less advanced stage if the amount of TrkB-FL is higher than the reference amount and/or the amount of TrkB-ICD is lower than the reference amount.

In an embodiment, the reference amount may be representative of severe AD, wherein the measured amount of TrkB-FL and/or TrkB-ICD in a sample obtained from a subject is indicative of the subject having AD at an even more advanced stage if the amount of TrkB-FL is lower than the reference amount or the amount of TrkB-ICD is higher than the reference amount. In an embodiment, the reference amount may be representative of severe AD, wherein the measured amount of TrkB-FL and/or TrkB-ICD in a sample obtained from a subject is indicative of the subject having severe AD if the amount of TrkB-FL and/or TrkB-ICD is the same or similar to the reference amount. In an embodiment, the reference amount may be representative of severe AD, wherein the measured amount of TrkB-FL and/or TrkB-ICD in a sample obtained from a subject is indicative of the subject not having AD or having AD at a less advanced stage if the amount of TrkB-FL is higher than the reference amount and/or the amount of TrkB-ICD is lower than the reference amount.

In an embodiment, there is disclosed a method for diagnosing AD or for determining the stage of AD, comprising: i) measuring the amount of TrkB-FL and/or TrkB-T' in a sample obtained from a subject, and ii) comparing the amount of TrkB-FL and/or TrkB-T' to a reference amount.

In an embodiment, there is disclosed a method for diagnosing AD or for determining the stage of AD, comprising comparing the amount of TrkB-FL and/or TrkB-T' measured in a sample obtained from a subject to a reference amount.

A lower amount of TrkB-FL in the sample obtained from the subject in comparison to the reference amount may be indicative of AD or AD at a more advanced stage than that represented by the reference amount. The same or a similar amount of TrkB-FL may be indicative of AD at the same stage as that represented by the reference amount or of no AD if the reference represents a patient without AD. A higher amount of TrkB-FL in comparison to a reference amount may be indicative of no AD or AD at a less advanced stage than that represented by the reference amount. A higher amount of TrkB-T' in the sample obtained from the subject in comparison to a reference amount may be indicative of AD or AD at a more advanced stage than that represented by the reference amount. The same or a similar amount of TrkB-T' may be indicative of AD at the same stage as that represented by the reference amount or of no AD if the reference represents a patient without AD. A lower amount of TrkB-T' in comparison to a reference amount may be indicative of no AD or AD at a less advanced stage than that represented by the reference amount. Hence, the method allows the determination of whether a subject has AD and/or of the stage of AD.

The reference amount may be representative of the amount of TrkB-FL and/orTrkB-T' in a subject without AD. The reference amount may be representative of the amount of TrkB-FL and/or TrkB-T' in a subject with mild AD. The reference amount may be representative of the amount of TrkB-FL and/orTrkB-T' in a subject with moderate AD. The reference amount may be representative of the amount of TrkB-FL and/or TrkB-T' in a subject with severe AD. The reference amount may have been determined from the same subject before disease onset, after diagnosis of AD, after diagnosis of mild AD, after diagnosis with moderate AD, or after diagnosis with severe AD. The reference amount may be obtained from a one or more, or a plurality of subjects. The reference amount may have been calculated from pre-collected data or from pre-collected samples. In some embodiments, the reference amount may be adjusted to take into account the age, sex, weight, ethnicity, amount of physical exercise, or other relevant factor of the subject to be tested. The reference amount may represent or take into account the natural variations of the biomarkers found in the representative population, and hence the reference value may be a range.

In an embodiment, the reference amount may be representative of mild AD, wherein the measured amount of TrkB-FL and/or TrkB-T' in a sample obtained from a subject is indicative of the subject having AD at a more advanced stage if the amount of TrkB-FL is lower than the reference amount and/or the amount of TrkB-T' is higher than the reference amount. In an embodiment, the reference amount may be representative of mild AD, wherein the measured amount of TrkB-FL and/or TrkB-T' in a sample obtained from a subject is indicative of the subject having mild AD if the amount of TrkB-FL and/or TrkB-T' is the same or similar to the reference amount. In an embodiment, the reference amount may be representative of mild AD, wherein the measured amount of TrkB-FL and/orTrkB-T' in a sample obtained from a subject is indicative of the subject not having AD or having AD at a less advanced stage if the amount of TrkB-FL is higher than the reference amount and/or the amount of TrkB-T' is lower than the reference amount.

In an embodiment, the reference amount may be representative of moderate AD, wherein the measured amount of TrkB-FL and/or TrkB- TrkB-T' in a sample obtained from a subject is indicative of the subject having AD at a more advanced stage if the amount of TrkB-FL is lower than the reference amount and/or the amount of TrkB-T' is higher than the reference amount. In an embodiment, the reference amount may be representative of moderate AD, wherein the measured amount of TrkB-FL and/or TrkB-T' in a sample obtained from a subject is indicative of the subject having moderate AD if the amount of TrkB-FL and/or TrkB-T' is the same or similar to the reference amount. In an embodiment, the reference amount may be representative of moderate AD, wherein the measured amount of TrkB-FL and/or TrkB-T' in a sample obtained from a subject is indicative of the subject not having AD or having AD at a less advanced stage if the amount of TrkB-FL is higher than the reference amount and/or the amount of TrkB-T' is lower than the reference amount.

In an embodiment, the reference amount may be representative of severe AD, wherein the measured amount of TrkB-FL and/or TrkB-T' in a sample obtained from a subject is indicative of the subject having AD at an even more advanced stage if the amount of TrkB-FL is lower than the reference amount and/or the amount of TrkB-T' is higher than the reference amount. In an embodiment, the reference amount may be representative of severe AD, wherein the measured amount of TrkB-FL and/or TrkB-T' in a sample obtained from a subject is indicative of the subject having severe AD if the amount of TrkB-FL and/or TrkB-T' is the same or similar to the reference amount. In an embodiment, the reference amount may be representative of severe AD, wherein the measured amount of TrkB-FL and/or TrkB-T' in a sample obtained from a subject is indicative of the subject not having AD or having AD at a less advanced stage if the amount of TrkB-FL is higher than the reference amount and/or the amount of TrkB-T' is lower than the reference amount.

In another embodiment, there is disclosed a method for diagnosing AD or for determining the stage of AD, comprising: i) measuring the amount of TrkB-FL and TrkB-ICD in a sample obtained from a subject, ii) calculating the TrkB-ICD:TrkB-FL ratio, and iii) comparing the TrkB-ICD:TrkB-FL ratio to a reference value.

In another embodiment, there is disclosed a method for diagnosing AD or for determining the stage of AD, comprising comparing the TrkB-ICD:TrkB-FL ratio measured in a sample obtained from a subject to a reference value.

A higher TrkB-ICD:TrkB-FL ratio in comparison to the reference value may be indicative of AD or AD at a more advanced stage than that represented by the reference value. The same or a similar TrkB-ICD:TrkB-FL ratio in comparison to the reference value may be indicative of AD at the same stage as that represented by the reference value or of no AD if the reference represents a patient without AD. A lower TrkB-ICD:TrkB-FL ratio in comparison to the reference value may be indicative of no AD or AD at a less advanced stage than that represented by the reference value. Hence, the method allows the determination of whether subject has AD and/or of the stage of AD.

The reference value may be representative of the TrkB-ICD:TrkB-FL ratio in a subject without AD. The reference value may be representative of the TrkB-ICD:TrkB-FL ratio in a subject with mild AD. The reference value may be representative of the TrkB-ICD:TrkB-FL ratio in a subject with moderate AD. The reference value may be representative of the TrkB-ICD:TrkB-FL ratio in a subject with severe AD. The reference value may have been determined from the same subject before disease onset, after diagnosis of AD, after diagnosis of mild AD, after diagnosis with moderate AD, or after diagnosis with severe AD. The reference value may be obtained from a one or more, or a plurality of subjects. The reference value may have been calculated from pre-collected data or from pre-collected samples. In some embodiments, the reference value may be adjusted to take into account the age, sex, weight, ethnicity, amount of physical exercise, or other relevant factor of the subject to be tested. The reference value may represent or take into account the natural variations of the biomarkers found in the representative population, and hence the reference value may be a range.

In some embodiments, a subject is diagnosed with AD if the subject's measured TrkB-ICD:TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to the TrkB-ICD:TrkB-FL ratio measured for subjects without AD or a representative reference value. For instance, a subject with a measured TrkB-ICD:TrkB-FL ratio of 4:1 would have a ratio that is increased by 100% in comparison to a representative value of 2:1. In some embodiments, a subject is diagnosed with AD if the subject's measured TrkB-ICD:TrkB-FL ratio is increased by at least 200% in comparison to the TrkB-ICD:TrkB-FL ratio measured for subjects without AD or a representative reference value. In some embodiments, a subject is diagnosed with AD if the subject's measured TrkB-ICD:TrkB-FL ratio is increased by at least 300% in comparison to the TrkB-ICD:TrkB-FL ratio measured for subjects without AD or a representative reference value. Hence, the method allows the determination of whether subject has AD based upon the subject's measured TrkB-ICD:TrkB-FL ratio.

In some embodiments, the threshold at which a subject is diagnosed with AD depends on the sample being tested. In some embodiments, the subject's TrkB-ICD:TrkB-FL ratio is measured in a plasma sample, and the subject is diagnosed with AD if the measured TrkB-ICD:TrkB-FL ratio is increased by at least 10%, at least 20%, at least 30%, or at least 40% in comparison to the TrkB-ICD:TrkB-FL ratio measured for subjects without AD or a representative reference value. In a particular embodiment, the subject is diagnosed with AD if the measured TrkB-ICD:TrkB-FL ratio is increased by at least 20% in comparison to the TrkB-ICD:TrkB-FL ratio measured for subjects without AD or a representative reference value.

In some embodiments, the threshold at which a subject is diagnosed with AD depends on the sample being tested. In some embodiments, the subject's TrkB-ICD:TrkB-FL ratio is measured in a CSF sample, and the subject is diagnosed with AD if the measured TrkB-ICD:TrkB-FL ratio is increased by at least 20%, at least 30%, at least 40%, or at least 50% in comparison to the TrkB-ICD:TrkB-FL ratio measured for subjects without AD or a representative reference value. In a particular embodiment, the subject is diagnosed with AD if the measured TrkB-ICD:TrkB-FL ratio is increased by at least 40% in comparison to the TrkB-ICD:TrkB-FL ratio measured for subjects without AD or a representative reference value.

In an embodiment, the reference value may be representative of mild AD, wherein the calculated TrkB-ICD:TrkB-FL ratio in a sample obtained from a subject is indicative of the subject having AD at a more advanced stage if the calculated TrkB-ICD:TrkB-FL ratio is higher than the reference value. In an embodiment, the reference value may be representative of mild AD, wherein the calculated TrkB-ICD:TrkB-FL ratio in a sample obtained from a subject is indicative of the subject having mild AD if the calculated TrkB-ICD:TrkB-FL ratio is the same or similar to the reference value. In an embodiment, the reference value may be representative of mild AD, wherein the calculated TrkB-ICD:TrkB-FL ratio in a sample obtained from a subject is indicative of the subject not having AD or having AD at a less advanced stage if the calculated TrkB-ICD:TrkB-FL ratio is lower than the reference value.

In an embodiment, the reference value may be representative of moderate AD, wherein the calculated TrkB-ICD:TrkB-FL ratio in a sample obtained from a subject is indicative of the subject having AD at a more advanced stage if the calculated TrkB-ICD:TrkB-FL ratio is higher than the reference value. In an embodiment, the reference value may be representative of moderate AD, wherein the calculated TrkB-ICD:TrkB-FL ratio in a sample obtained from a subject is indicative of the subject having moderate AD if the calculated TrkB-ICD:TrkB-FL ratio is the same or similar to the reference value. In an embodiment, the reference value may be representative of moderate AD, wherein the calculated TrkB-ICD:TrkB-FL ratio in a sample obtained from a subject is indicative of the subject not having AD or having AD at a less advanced stage if the calculated TrkB-ICD:TrkB-FL ratio is lower than the reference value.

In an embodiment, the reference value may be representative of severe AD, wherein the calculated TrkB-ICD:TrkB-FL ratio in a sample obtained from a subject is indicative of the subject having AD at an even more advanced stage if the calculated TrkB-ICD:TrkB-FL ratio is higher than the reference value. In an embodiment, the reference value may be representative of severe AD, wherein the calculated TrkB-ICD:TrkB-FL ratio in a sample obtained from a subject is indicative of the subject having severe AD if the calculated TrkB-ICD:TrkB-FL ratio is the same or similar to the reference value. In an embodiment, the reference value may be representative of severe AD, wherein the calculated TrkB-ICD:TrkB-FL ratio in a sample obtained from a subject is indicative of the subject not having AD or having AD at a less advanced stage if the calculated TrkB-ICD:TrkB-FL ratio is lower than the reference value.

In an embodiment, the subject to be tested may have mild cognitive impairment. In another embodiment, the subject to be tested may have cognitive impairment. In other embodiments, the subject to be tested may have mild, moderate, or severe AD.

In another embodiment, there is disclosed a method for diagnosing AD or for determining the stage of AD, comprising: i) measuring the amount of TrkB-FL and TrkB-T' in a sample obtained from a subject, ii) calculating the TrkB-T':TrkB-FL ratio, and iii) comparing the TrkB-T':TrkB-FL ratio to a reference value.

In another embodiment, there is disclosed a method for diagnosing AD or for determining the stage of AD, comprising comparing the TrkB-T':TrkB-FL ratio measured in a sample obtained from a subject to a reference value.

A higher TrkB-T':TrkB-FL ratio in comparison to the reference value may be indicative of AD or AD at a more advanced stage than that represented by the reference value. The same or a similar TrkB-T':TrkB-FL ratio in comparison to the reference value may be indicative of AD at the same stage as that represented by the reference value or of no AD if the reference represents a patient without AD. A lower TrkB-T':TrkB-FL ratio in comparison to the reference value may be indicative of no AD or AD at a less advanced stage than that represented by the reference value. Hence, the method allows the determination of whether subject has AD and/or of the stage of AD.

The reference value may be representative of the TrkB-T':TrkB-FL ratio in a subject without AD. The reference value may be representative of the TrkB-T':TrkB-FL ratio in a subject with mild AD. The reference value may be representative of the TrkB-T':TrkB-FL ratio in a subject with moderate AD. The reference value may be representative of the TrkB-T':TrkB-FL ratio in a subject with severe AD. The reference value may have been determined from the same subject before disease onset, after diagnosis of AD, after diagnosis of mild AD, after diagnosis with moderate AD, or after diagnosis with severe AD. The reference value may be obtained from a one or more, or a plurality of subjects. The reference value may have been calculated from pre-collected data or from pre-collected samples. In some embodiments, the reference value may be adjusted to take into account the age, sex, weight, ethnicity, amount of physical exercise, or other relevant factor of the subject to be tested. The reference value may represent or take into account the natural variations of the biomarkers found in the representative population, and hence the reference value may be a range.

In some embodiments, a subject is diagnosed with AD if the subject's measured TrkB-T':TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to the TrkB-T':TrkB-FL ratio measured for subjects without AD or a representative reference value. For instance, a subject with a measured TrkB-T':TrkB-FL ratio of 4:1 would have a ratio that is increased by 100% in comparison to a representative value of 2:1. In some embodiments, a subject is diagnosed with AD if the subject's measured TrkB-T':TrkB-FL ratio is increased by at least 200% in comparison to the TrkB-T':TrkB-FL ratio measured for subjects without AD or a representative reference value. In some embodiments, a subject is diagnosed with AD if the subject's measured TrkB-T':TrkB-FL ratio is increased by at least 300% in comparison to the TrkB-T':TrkB-FL ratio measured for subjects without AD or a representative reference value. Hence, the method allows the determination of whether subject has AD based upon the subject's measured TrkB-T':TrkB-FL ratio.

In some embodiments, the threshold at which a subject is diagnosed with AD depends on the sample being tested. In some embodiments, the subject's TrkB-T':TrkB-FL ratio is measured in a plasma sample, and the subject is diagnosed with AD if the measured TrkB-T':TrkB-FL ratio is increased by at least 10%, at least 20%, at least 30%, or at least 40% in comparison to the TrkB-T':TrkB-FL ratio measured for subjects without AD or a representative reference value. In a particular embodiment, the subject is diagnosed with AD if the measured TrkB-T':TrkB-FL ratio is increased by at least 20% in comparison to the TrkB-T':TrkB-FL ratio measured for subjects without AD or a representative reference value.

In some embodiments, the threshold at which a subject is diagnosed with AD depends on the sample being tested. In some embodiments, the subject's TrkB-T':TrkB-FL ratio is measured in a CSF sample, and the subject is diagnosed with AD if the measured TrkB-T':TrkB-FL ratio is increased by at least 20%, at least 30%, at least 40%, or at least 50% in comparison to the TrkB-T':TrkB-FL ratio measured for subjects without AD or a representative reference value. In a particular embodiment, the subject is diagnosed with AD if the measured TrkB-T':TrkB-FL ratio is increased by at least 40% in comparison to the TrkB-T':TrkB-FL ratio measured for subjects without AD or a representative reference value.

In an embodiment, the reference value may be representative of mild AD, wherein the calculated TrkB-T':TrkB-FL ratio in a sample obtained from a subject is indicative of the subject having AD at a more advanced stage if the calculated TrkB-T':TrkB-FL ratio is higher than the reference value. In an embodiment, the reference value may be representative of mild AD, wherein the calculated TrkB-T':TrkB-FL ratio in a sample obtained from a subject is indicative of the subject having mild AD if the calculated TrkB-T':TrkB-FL ratio is the same or similar to the reference value. In an embodiment, the reference value may be representative of mild AD, wherein the calculated TrkB-T':TrkB-FL ratio in a sample obtained from a subject is indicative of the subject not having AD or having AD at a less advanced stage if the calculated TrkB-T':TrkB-FL ratio is lower than the reference value.

In an embodiment, the reference value may be representative of moderate AD, wherein the calculated TrkB-T':TrkB-FL ratio in a sample obtained from a subject is indicative of the subject having AD at a more advanced stage if the calculated TrkB-T':TrkB-FL ratio is higher than the reference value. In an embodiment, the reference value may be representative of moderate AD, wherein the calculated TrkB-T':TrkB-FL ratio in a sample obtained from a subject is indicative of the subject having moderate AD if the calculated TrkB-T':TrkB-FL ratio is the same or similar to the reference value. In an embodiment, the reference value may be representative of moderate AD, wherein the calculated TrkB-T':TrkB-FL ratio in a sample obtained from a subject is indicative of the subject not having AD or having AD at a less advanced stage if the calculated TrkB-T':TrkB-FL ratio is lower than the reference value.

In an embodiment, the reference value may be representative of severe AD, wherein the calculated TrkB-T':TrkB-FL ratio in a sample obtained from a subject is indicative of the subject having AD at an even more advanced stage if the calculated TrkB-T':TrkB-FL ratio is higher than the reference value. In an embodiment, the reference value may be representative of severe AD, wherein the calculated TrkB-T':TrkB-FL ratio in a sample obtained from a subject is indicative of the subject having severe AD if the calculated TrkB-T':TrkB-FL ratio is the same or similar to the reference value. In an embodiment, the reference value may be representative of severe AD, wherein the calculated TrkB-T':TrkB-FL ratio in a sample obtained from a subject is indicative of the subject not having AD or having AD at a less advanced stage if the calculated TrkB-T':TrkB-FL ratio is lower than the reference value.

In an embodiment, the subject to be tested may have mild cognitive impairment. In another embodiment, the subject to be tested may have cognitive impairment. In other embodiments, the subject to be tested may have mild, moderate, or severe AD.

In an embodiment, there is disclosed a method for diagnosing AD, comprising: i) measuring the amount of TrkB-FL and TrkB-ICD in a sample obtained from a subject with mild cognitive impairment, ii) calculating the TrkB-ICD:TrkB-FL ratio, and iii) comparing the calculated TrkB-ICD:TrkB-FL ratio to a reference value obtained from at least one subject without Alzheimer's disease, wherein a calculated TrkB-ICD:TrkB-FL ratio increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to the reference value is indicative of AD. In an embodiment, a calculated TrkB-ICD:TrkB-FL ratio that is not increased or is increased by no more than 5%, no more than 10%, or no more than 15% in comparison to said reference value may indicate that the subject does not have AD. Hence, the method allows the determination of whether the subject with mild cognitive impairment has AD.

In an embodiment, there is disclosed a method for diagnosing AD, comprising: i) measuring the amount of TrkB-FL and TrkB-T' in a sample obtained from a subject with mild cognitive impairment, ii) calculating the TrkB-T':TrkB-FL ratio, and iii) comparing the calculated TrkB-T':TrkB-FL ratio to a reference value obtained from at least one subject without Alzheimer's disease, wherein a calculated TrkB-T':TrkB-FL ratio increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to the reference value is indicative of AD. In an embodiment, a calculated TrkB-T':TrkB-FL ratio that is not increased or is increased by no more than 5%, no more than 10%, or no more than 15% in comparison to said reference value may indicate that the subject does not have AD. Hence, the method allows the determination of whether the subject with mild cognitive impairment has AD.

Also disclosed are methods of treatment comprising: i) any of the methods for determining the stage of AD disclosed herein to identify a patient as having mild AD, and ii) administering a treatment to said patient. The treatment may comprise the administration of any pharmaceutical composition, polypeptide, nucleic acid, genetic construct, vector, or cell of the parent application EP'999. The treatment may comprise the administration of cholinesterase inhibitors, for instance donepezil, rivastigmine, or galantamine. The treatment might comprise the administration of choline alphoscerate, idebenone (Ideben), piracetam, quetiapine fumarate (Qutadac), trifluperidol (Triperidol), and/or citicoline.

Further disclosed are methods of treatment comprising: i) any of the methods for determining the stage of AD disclosed herein to identify a patient as having moderate AD, and ii) administering a treatment to said patient. The treatment may comprise the administration of any pharmaceutical composition, polypeptide, nucleic acid, genetic construct, vector, or cell of the parent application EP'999. The treatment may comprise the administration of cholinesterase inhibitors, for instance donepezil, rivastigmine, or galantamine. The treatment may comprise the administration of an NMDA antagonist, for instance memantine. The treatment may comprise the administration of both a cholinesterase inhibitor and an NMDA antagonist. The treatment might comprise the administration of choline alphoscerate, idebenone (Ideben), piracetam, quetiapine fumarate (Qutadac), trifluperidol (Triperidol), and/or citicoline.

Further disclosed are methods of treatment comprising: i) any of the methods for determining the stage of AD disclosed herein to identify a patient as having severe AD, and ii) administering a treatment to said patient. The treatment may comprise the administration of any pharmaceutical composition, polypeptide, nucleic acid, genetic construct, vector, or cell of the parent application EP'999. The treatment may comprise the administration of cholinesterase inhibitors, for instance donepezil. The treatment may comprise the administration of an NMDA antagonist, for instance memantine. The treatment may comprise the administration of both a cholinesterase inhibitor and an NMDA antagonist. The treatment might comprise the administration of choline alphoscerate, idebenone (Ideben), piracetam, quetiapine fumarate (Qutadac), trifluperidol (Triperidol), and/or citicoline.

In other embodiments, the methods of diagnosis and the methods for determining the stage of a disease which comprise the use of TrkB-FL, TrkB-ICD, TrkB-T', the TrkB-T':TrkB-FL ratio, or the TrkB-ICD:TrkB-FL ratio as a biomarker, may be applied to any pathology characterized by excitotoxicity, epilepsy, prion-like diseases, muscular dystrophies, Huntington's disease, Parkinson's disease, multiple sclerosis, ischemia, stroke, brain trauma, any pathology related to TrkB-FL cleavage, depression, anxiety, bipolar disorders, schizophrenia, obesity, autism, rett syndrome, retinal ganglion cell loss, ageing, amyotrophic lateral sclerosis (Lou Gehrig's disease), the adverse neurologic complications of Down syndrome, diabetic peripheral neuropathy, or other types of peripheral neuropathy/.

For any of the methods of diagnosis and any of the methods for determining the stage of a disease disclosed herein, the sample in which the biomarkers are quantified may be CSF, blood, plasma, serum, or saliva.

The measurement of TrkB-FL, TrkB-T', and/or TrkB-ICD may be *in vitro.* TrkB-FL, TrkB-T', and/or TrkB-ICD may be measured by mass spectrometry, Western Blotting, or other methods suitable for the identification and quantification of specific proteins or peptides.

In some embodiments, the methods disclosed herein for diagnosing AD or for determining the stage of AD may be used to monitor the efficacy of a treatment. In particular embodiments, the methods disclosed herein for diagnosing AD or for determining the stage of AD may be used to monitor the efficacy of a treatment disclosed herein.

In another aspect of the invention, there is provided the use of TrkB-FL, TrkB-ICD, TrkB-T', the TrkB-T':TrkB-FL ratio, or the TrkB-ICD:TrkB-FL ratio for monitoring the efficacy of a treatment with an agent.

In an embodiment, there is disclosed a method for monitoring the efficacy of a treatment with an agent, comprising: i) measuring the amount of TrkB-FL and/or TrkB-ICD in a first sample obtained from a subject, ii) administering an agent to said subject, and iii) measuring the amount of TrkB-FL and/or TrkB-ICD in a second sample obtained from the subject.

In an embodiment, there is disclosed a method for monitoring the efficacy of a treatment with an agent, comprising: i) measuring the amount of TrkB-FL and/or TrkB-ICD in a first sample obtained from a subject, and ii) measuring the amount of TrkB-FL and/or TrkB-ICD in a second sample obtained from the subject after administration of an agent to the subject.

In an embodiment, there is disclosed a method for monitoring the efficacy of a treatment with an agent, comprising comparing the amount of TrkB-FL and/or TrkB-ICD measured in a first sample obtained from a subject to the amount of TrkB-FL and/or TrkB-ICD measured in a second sample obtained from the subject after administration of an agent to the subject.

In some embodiments, if the amount of TrkB-FL measured in the second sample is higher than the amount of TrkB-FL measured in the first sample, this may be indicative that treatment with the agent is efficacious. In some embodiments, if the amount of TrkB-FL measured in the second sample is the same or similar to the amount of TrkB-FL measured in the first sample, this may be indicative that treatment with the agent has delayed disease progression. In some embodiments, if the amount of TrkB-FL measured in the second sample is lower than the amount of TrkB-FL measured in the first sample, this may be indicative that treatment with the agent is not efficacious. Hence, the method allows the determination of whether a treatment has been efficacious.

In some embodiments, the amount ofTrkB-FL measured in the second sample may be compared to an expected amount of TrkB-FL. The expected amount of TrkB-FL may be based upon the amount of TrkB-FL measured in the first sample and adjusted by allowing for the expected disease progression for an untreated patient during the period between obtaining the first sample and obtaining the second sample. In such embodiments, if the amount of TrkB-FL measured in the second sample is higher than the expected amount of TrkB-FL, this may be indicative that treatment with the agent is efficacious. If the amount of TrkB-FL measured in the second sample is the same or similar to the expected amount of TrkB-FL, this may be indicative that treatment with the agent with the agent is not efficacious. If the amount of TrkB-FL measured in the second sample is lower than the expected amount of TrkB-FL, this may be indicative that treatment with the agent is not efficacious. Hence, the method allows the determination of whether a treatment has been efficacious.

In some embodiments, if the amount of TrkB-ICD measured in the second sample is higher than the amount of TrkB-ICD measured in the first sample, this may be indicative that treatment with the agent is not efficacious. In some embodiments, if the amount of TrkB-ICD measured in the second sample is the same or similar to the amount of TrkB-ICD measured in the first sample, this may be indicative that treatment with the agent has delayed disease progression. In some embodiments, if the amount of TrkB-ICD measured in the second sample is lower than the amount of TrkB-ICD measured in the first sample, this may be indicative that treatment with the agent is efficacious. Hence, the method allows the determination of whether a treatment has been efficacious.

In some embodiments, the amount of TrkB-ICD measured in the second sample may be compared to an expected amount of TrkB-ICD. The expected amount of TrkB-ICD may be based upon the amount of TrkB-ICD measured in the first sample and adjusted by allowing for the expected disease progression for an untreated patient during the period between obtaining the first sample and obtaining the second sample. In such embodiments, if the amount of TrkB-ICD measured in the second sample is higher than the expected amount of TrkB-ICD, this may be indicative that treatment with the agent is not efficacious. If the amount of TrkB-ICD measured in the second sample is the same or similar to the expected amount of TrkB-ICD, this may be indicative that treatment with the agent with the agent is not efficacious. If the amount of TrkB-ICD measured in the second sample is lower than the expected amount of TrkB-ICD, this may be indicative that treatment with the agent is efficacious. Hence, the method allows the determination of whether a treatment has been efficacious.

In an embodiment, there is disclosed a method for monitoring the efficacy of a treatment with an agent, comprising: i) measuring the amount of TrkB-FL and/or TrkB-T' in a first sample obtained from a subject, ii) administering an agent to said subject, and iii) measuring the amount of TrkB-FL and/or TrkB-T' in a second sample obtained from the subject.

In an embodiment, there is disclosed a method for monitoring the efficacy of a treatment with an agent, comprising: i) measuring the amount of TrkB-FL and/or TrkB-T' in a first sample obtained from a subject, and ii) measuring the amount of TrkB-FL and/or TrkB-T' in a second sample obtained from the subject after administration of an agent to the subject.

In an embodiment, there is disclosed a method for monitoring the efficacy of a treatment with an agent, comprising comparing the amount of TrkB-FL and/orTrkB-T' measured in a first sample obtained from a subject to the amount of TrkB-FL and/or TrkB-T' measured in a second sample obtained from the subject after administration of an agent to the subject.

In some embodiments, if the amount of TrkB-FL measured in the second sample is higher than the amount of TrkB-FL measured in the first sample, this may be indicative that treatment with the agent is efficacious. In some embodiments, if the amount of TrkB-FL measured in the second sample is the same or similar to the amount of TrkB-FL measured in the first sample, this may be indicative that treatment with the agent has delayed disease progression. In some embodiments, if the amount of TrkB-FL measured in the second sample is lower than the amount of TrkB-FL measured in the first sample, this may be indicative that treatment with the agent is not efficacious. Hence, the method allows the determination of whether a treatment has been efficacious.

In some embodiments, the amount ofTrkB-FL measured in the second sample may be compared to an expected amount of TrkB-FL. The expected amount of TrkB-FL may be based upon the amount of TrkB-FL measured in the first sample and adjusted by allowing for the expected disease progression for an untreated patient during the period between obtaining the first sample and obtaining the second sample. In such embodiments, if the amount of TrkB-FL measured in the second sample is higher than the expected amount of TrkB-FL, this may be indicative that treatment with the agent is efficacious. If the amount of TrkB-FL measured in the second sample is the same or similar to the expected amount of TrkB-FL, this may be indicative that treatment with the agent with the agent is not efficacious. If the amount of TrkB-FL measured in the second sample is lower than the expected amount of TrkB-FL, this may be indicative that treatment with the agent is not efficacious. Hence, the method allows the determination of whether a treatment has been efficacious.

In some embodiments, if the amount of TrkB-T' measured in the second sample is higher than the amount of TrkB-T' measured in the first sample, this may be indicative that treatment with the agent is not efficacious. In some embodiments, if the amount of TrkB-T' measured in the second sample is the same or similar to the amount of TrkB-T' measured in the first sample, this may be indicative that treatment with the agent has delayed disease progression. In some embodiments, if the amount of TrkB-T' measured in the second sample is lower than the amount of TrkB-T' measured in the first sample, this may be indicative that treatment with the agent is efficacious. Hence, the method allows the determination of whether a treatment has been efficacious.

In some embodiments, the amount of TrkB-T' measured in the second sample may be compared to an expected amount of TrkB-T'. The expected amount of TrkB-T' may be based upon the amount of TrkB-T' measured in the first sample and adjusted by allowing for the expected disease progression for an untreated patient during the period between obtaining the first sample and obtaining the second sample. In such embodiments, if the amount of TrkB-T' measured in the second sample is higher than the expected amount of TrkB-T', this may be indicative that treatment with the agent is not efficacious. If the amount of TrkB-T' measured in the second sample is the same or similar to the expected amount of TrkB-T', this may be indicative that treatment with the agent with the agent is not efficacious. If the amount of TrkB-T' measured in the second sample is lower than the expected amount of TrkB-T', this may be indicative that treatment with the agent is efficacious. Hence, the method allows the determination of whether a treatment has been efficacious.

In an embodiment, there is disclosed a method for monitoring the efficacy of a treatment with an agent, comprising: i) measuring the amount of TrkB-FL and TrkB-ICD in a first sample obtained from a subject, ii) calculating the TrkB-ICD:TrkB-FL ratio in said first sample, iii) administering an agent to said subject, iv) measuring the amount of TrkB-FL and TrkB-ICD in a second sample obtained from said subject, and v) calculating the TrkB-ICD:TrkB-FL ratio in said second sample.

In an embodiment, there is disclosed a method for monitoring the efficacy of a treatment with an agent, comprising: i) calculating the TrkB-ICD:TrkB-FL ratio in a first sample obtained from a subject, and ii) calculating the TrkB-ICD:TrkB-FL ratio a second sample obtained from a subject after administration of an agent to the subject.

In an embodiment, there is disclosed a method for monitoring the efficacy of a treatment with an agent, comprising comparing the TrkB-ICD:TrkB-FL ratio measured in a first sample obtained from a subject to the TrkB-ICD:TrkB-FL ratio measured in a second sample obtained from the subject after administration of an agent to the subject.

In some embodiments, if the TrkB-ICD:TrkB-FL ratio measured in the second sample is higher than the TrkB-ICD:TrkB-FL ratio measured in the first sample, this may be indicative that treatment with the agent is not efficacious. In some embodiments, if the TrkB-ICD:TrkB-FL ratio measured in the second sample is the same or similar to the TrkB-ICD:TrkB-FL ratio measured in the first sample, this may be indicative that treatment with the agent is efficacious and has delayed disease progression. In some embodiments, if the TrkB-ICD:TrkB-FL ratio measured in the second sample is lower than the TrkB-ICD:TrkB-FL ratio measured in the first sample, this may be indicative that treatment with the agent is efficacious. Hence, the method may allow the determination of whether a treatment has been efficacious.

In some embodiments, the TrkB-ICD:TrkB-FL ratio measured in the second sample may be compared to an expected TrkB-ICD:TrkB-FL ratio. The expected TrkB-ICD:TrkB-FL ratio may be based upon the TrkB-ICD:TrkB-FL ratio measured in the first sample and adjusted by allowing for the expected disease progression for an untreated patient during the period between obtaining the first sample and obtaining the second sample. In such embodiments, if the TrkB-ICD:TrkB-FL ratio measured in the second sample is higher than the expected TrkB-ICD:TrkB-FL ratio, this may be indicative that treatment with the agent is not efficacious. If the TrkB-ICD:TrkB-FL ratio measured in the second sample is the same or similar to the expected TrkB-ICD:TrkB-FL ratio, this may be indicative that treatment with the agent with the agent is not efficacious. If the TrkB-ICD:TrkB-FL ratio measured in the second sample is lower than the expected TrkB-ICD:TrkB-FL ratio, this may be indicative that treatment with the agent is efficacious. Hence, the method may allow the determination of whether a treatment has been efficacious.

In an embodiment, there is disclosed a method for monitoring the efficacy of a treatment with an agent, comprising: i) measuring the amount of TrkB-FL and TrkB-T' in a first sample obtained from a subject, ii) calculating the TrkB-T':TrkB-FL ratio in said first sample, iii) administering an agent to said subject, iv) measuring the amount of TrkB-FL and TrkB-T' in a second sample obtained from said subject, and v) calculating the TrkB-T':TrkB-FL ratio in said second sample.

In an embodiment, there is disclosed a method for monitoring the efficacy of a treatment with an agent, comprising: i) calculating the TrkB-T':TrkB-FL ratio in a first sample obtained from a subject, and ii) calculating the TrkB-T':TrkB-FL ratio a second sample obtained from a subject after administration of an agent to the subject.

In an embodiment, there is disclosed a method for monitoring the efficacy of a treatment with an agent, comprising comparing the TrkB-T':TrkB-FL ratio measured in a first sample obtained from a subject to the TrkB-T':TrkB-FL ratio measured in a second sample obtained from the subject after administration of an agent to the subject.

In some embodiments, if the TrkB-T':TrkB-FL ratio measured in the second sample is higher than the TrkB-T':TrkB-FL ratio measured in the first sample, this may be indicative that treatment with the agent is not efficacious. In some embodiments, if the TrkB-T':TrkB-FL ratio measured in the second sample is the same or similar to the TrkB-T':TrkB-FL ratio measured in the first sample, this may be indicative that treatment with the agent has delayed disease progression. In some embodiments, if the TrkB-T':TrkB-FL ratio measured in the second sample is lower than the TrkB-T':TrkB-FL ratio measured in the first sample, this may be indicative that treatment with the agent is efficacious. Hence, the method may allow the determination of whether a treatment has been efficacious.

In some embodiments, the TrkB-T':TrkB-FL ratio measured in the second sample may be compared to an expected TrkB-T':TrkB-FL ratio. The expected TrkB-T':TrkB-FL ratio may be based upon the TrkB-T':TrkB-FL ratio measured in the first sample and adjusted by allowing for the expected disease progression for an untreated patient during the period between obtaining the first sample and obtaining the second sample. In such embodiments, if the TrkB-T':TrkB-FL ratio measured in the second sample is higher than the expected TrkB-T':TrkB-FL ratio, this may be indicative that treatment with the agent is not efficacious. If the TrkB-T':TrkB-FL ratio measured in the second sample is the same or similar to the expected TrkB-T':TrkB-FL ratio, this may be indicative that treatment with the agent with the agent is not efficacious. If the TrkB-T':TrkB-FL ratio measured in the second sample is lower than the expected TrkB-T':TrkB-FL ratio, this may be indicative that treatment with the agent is efficacious. Hence, the method may allow the determination of whether a treatment has been efficacious.

The aforementioned methods for monitoring the efficacy of a treatment with an agent may relate to methods of treating AD, in which case the method is performed on samples obtained from subjects with AD. In another embodiment, the methods for monitoring the efficacy of a treatment with an agent may relate to any method of treatment disclosed herein. In other embodiments, the methods for monitoring the efficacy of a treatment with an agent may relate to any method of treatment for a pathology characterized by excitotoxicity, epilepsy, prion-like diseases, muscular dystrophies, Huntington's disease, Parkinson's disease, multiple sclerosis, ischemia, stroke, brain trauma, a pathology related to TrkB-FL cleavage, depression, anxiety, bipolar disorders, schizophrenia, obesity, autism, rett syndrome, retinal ganglion cell loss, ageing, amyotrophic lateral sclerosis (Lou Gehrig's disease), the adverse neurologic complications of Down syndrome, diabetic peripheral neuropathy, or other types of peripheral neuropathy. In these embodiments, the method is performed on samples obtained from subjects with the relevant pathology.

In an embodiment, the agent is a therapeutic agent or composition, polypeptide, nucleic acid, vector, genetic construct, cell, or pharmaceutical composition as disclosed in parent application EP'999. In an embodiment, the agent is a pharmaceutical composition.

In an embodiment, there is disclosed a method for monitoring the efficacy of a treatment with an agent, comprising: i) measuring the amount of TrkB-FL and TrkB-ICD in a first sample obtained from a subject, ii) calculating the TrkB-ICD:TrkB-FL ratio in said first sample, iii) administering a pharmaceutical composition to said subject, iv) measuring the amount of TrkB-FL and TrkB-ICD in a second sample obtained from said subject, and v) calculating the TrkB-ICD:TrkB-FL ratio in said second sample.

In an embodiment, there is disclosed a method for monitoring the efficacy of a treatment with an agent, comprising: i) measuring the amount of TrkB-FL and/or TrkB-ICD in a first sample obtained from a subject, and ii) measuring the amount of TrkB-FL and/or TrkB-ICD in a second sample obtained from the subject after administration of a pharmaceutical composition.

In an embodiment, there is disclosed a method for monitoring the efficacy of a treatment with an agent, comprising: comparing the amount of TrkB-FL and/or TrkB-ICD measured in a first sample obtained from a subject to the amount of TrkB-FL and/or TrkB-ICD measured in a second sample obtained from the subject after administration of a pharmaceutical composition.

In an embodiment, there is disclosed a method for monitoring the efficacy of an AD treatment with an agent, comprising: i) measuring the amount of TrkB-FL and TrkB-ICD in a first sample obtained from a subject with AD, ii) calculating the TrkB-ICD:TrkB-FL ratio in said first sample, iii) administering a pharmaceutical composition to said subject, iv) measuring the amount of TrkB-FL and TrkB-ICD in a second sample obtained from said subject, and v) calculating the TrkB-ICD:TrkB-FL ratio in said second sample.

In an embodiment, there is disclosed a method for monitoring the efficacy of an AD treatment with an agent, comprising: i) measuring the amount of TrkB-FL and/or TrkB-ICD in a first sample obtained from a subject with AD, and ii) measuring the amount of TrkB-FL and/or TrkB-ICD in a second sample obtained from the subject after administration of a pharmaceutical composition.

In an embodiment, there is disclosed a method for monitoring the efficacy of an AD treatment with an agent, comprising comparing the amount of TrkB-FL and/or TrkB-ICD measured in a first sample obtained from a subject with AD to the amount of TrkB-FL and/or TrkB-ICD measured in a second sample obtained from the subject after administration of a pharmaceutical composition.

In an embodiment, there is disclosed a method for monitoring the efficacy of a treatment with an agent, comprising: i) measuring the amount of TrkB-FL and TrkB-T' in a first sample obtained from a subject, ii) calculating the TrkB-T':TrkB-FL ratio in said first sample, iii) administering a pharmaceutical composition of the parent application EP'999 to said subject, iv) measuring the amount of TrkB-FL and TrkB-T' in a second sample obtained from said subject, and v) calculating the TrkB-T':TrkB-FL ratio in said second sample.

In an embodiment, there is disclosed a method for monitoring the efficacy of a treatment with an agent, comprising: i) measuring the amount of TrkB-FL and/or TrkB-T' in a first sample obtained from a subject, and ii) measuring the amount of TrkB-FL and/or TrkB-T' in a second sample obtained from the subject after administration of a pharmaceutical composition.

In an embodiment, there is disclosed a method for monitoring the efficacy of a treatment with an agent, comprising comparing the amount of TrkB-FL and/orTrkB-T' measured in a first sample obtained from a subject to the amount of TrkB-FL and/or TrkB-T' measured in a second sample obtained from the subject after administration of a pharmaceutical composition.

In an embodiment, there is disclosed a method for monitoring the efficacy of an AD treatment with an agent, comprising: i) measuring the amount of TrkB-FL and TrkB-T' in a first sample obtained from a subject with AD, ii) calculating the TrkB-T':TrkB-FL ratio in said first sample, iii) administering a pharmaceutical composition to said subject, iv) measuring the amount of TrkB-FL and TrkB-T' in a second sample obtained from said subject, and v) calculating the TrkB-T':TrkB-FL ratio in said second sample.

In an embodiment, there is disclosed a method for monitoring the efficacy of an AD treatment with an agent, comprising: i) measuring the amount of TrkB-FL and/or TrkB-T' in a first sample obtained from a subject with AD, and ii) measuring the amount of TrkB-FL and/or TrkB-T' in a second sample obtained from the subject after administration of a pharmaceutical composition.

In an embodiment, there is disclosed a method for monitoring the efficacy of an AD treatment with an agent, comprising comparing the amount of TrkB-FL and/or TrkB-T' measured in a first sample obtained from a subject with AD to the amount of TrkB-FL and/or TrkB-T' measured in a second sample obtained from the subject after administration of a pharmaceutical composition.

For any of the methods for monitoring the efficacy of a treatment disclosed herein, the sample in which the biomarkers are quantified may be CSF, blood, plasma, serum, or saliva. The measurement of TrkB-FL, TrkB-T', and/or TrkB-ICD may be *in vitro.* TrkB-FL, TrkB-T', and/or TrkB-ICD may be measured by mass spectrometry, Western Blotting, or other methods suitable for the identification and quantification of specific proteins or peptides.

A "subject", as used herein, may be a vertebrate, mammal, or domestic animal. Hence, medicaments according to the invention may be used to treat any mammal, for example livestock (e.g. a horse, cow, sheep, or pig), pets (e.g. a cat, dog, rabbit, or guinea pig), a laboratory animal (e.g. a mouse or a rat), or may be used in other veterinary applications. In particular embodiments, the subject is a human being. The methods of diagnosis, methods for determining the stage of a disease, or the methods for monitoring the efficacy of a treatment may be applied to any mammal, for example livestock (e.g. a horse, cow, sheep, or pig), pets (e.g. a cat, dog, rabbit, or guinea pig), a laboratory animal (e.g. a mouse or a rat), or, in particular embodiments, a human being.

A "substitution", as used herein, is the substitution of a given amino acid in a peptide for another amino acid.

A "conservative substitution" or "conserved substitution", as used herein, is the substitution of a given amino acid in a peptide for another amino acid of similar characteristics. For instance, the amino acids may have similar charge, hydrophobicity, or size. The amino acids may be within the same class, for instance aliphatic, hydroxyl- or sulfur- containing, aromatic, basic, or acidic. Conservative substitutions are likely to be phenotypically silent. Examples of conservative substitutions may be within the group glycine, alanine, valine, leucine, and isoleucine; or within the group serine, cysteine, threonine, and methionine; or within the group phenylalanine, tyrosine, and tryptophan; or within the group histidine, lysine, and arginine; or within the group aspartate, glutamate, asparagine, and glutamine.

A "replacement", as used herein, is the replacement of a given amino acid in a peptide for another moiety.

"Modifications" as used herein can refer to post-translation modifications to amino acids; artificial modifications to result in non-naturally occurring amino acids; moieties added to naturally occurring amino acids; sugar residues or modified sugar residues added to amino acids or peptides; additional residues, moieties, or caps to the terminus or termini of peptides; or other modifications known in the art. The modifications may be for the purpose of increasing the stability of the agent, increasing the bioavailability of the agent, altering the charge or the agent, or for the achievement of other desired effects.

A "neuropathological condition", as used herein, may be any condition with causes harm or results in abnormal function of the nervous system tissue, for instance the brain, of a subject.

All publications, patent applications, patents, and other references mentioned herein are incorporated by reference. The references cited herein are not admitted to be prior art to the claimed application. In the case of conflict, the present specification, including definitions, will control.

In addition, the materials, methods, and examples are illustrative only and not intended to be limiting. Other features and advantages of the disclosure will become apparent from the following detailed description in conjunction with the examples.

### EXAMPLES

In Alzheimer's disease (AD) pathophysiology, Brain-derived Neurotrophic Factor (BDNF) signaling is impaired by Amyloid β (Aβ)-triggered cleavage of TrkB-FL, compromising neuronal survival, differentiation and synaptic physiology. Using post-mortem human brain samples of AD patients, the inventors demonstrate herein that TrkB-FL cleavage increases during AD progression. The inventors designed a small TAT-TrkB peptide that prevents this cleavage and restores BDNF physiological actions on synaptic transmission and plasticity.

### Changes in biomarkers during AD progression

The inventors evaluated TrkB-FL cleavage *in post-mortem* inferior temporal cortical samples from human patients diagnosed with AD, at different stages of disease progression. The inventors detected a significant decrease of TrkB-FL protein levels and an increase in the levels of TrkB-ICD fragment, as AD progressed (Fig. 1a-b). This observation was corroborated by Mass Spectrometry quantification of TrkB-FL (P<0.05; Fig.4a). TrkB-FL transcript levels did not significantly change with AD progression (P>0.05; Fig.1c) and post-mortem delay time until sample collection did not affect TrkB-FL cleavage (P>0.05; Fig.4b-c).

Overall, these data suggest that TrkB-FL cleavage may be potentially implicated in the mechanisms driving AD progression by (1) compromising canonical BDNF signaling and (2) favoring TrkB-ICD formation, which may propagate Aβ-triggered toxicity^{6,7}.

### Use of TrkB-FL and TrkB-ICD as AD biomarkers

The inventors demonstrated that in CSF samples collected from MCI patients diagnosed with AD there is a decrease of TrkB-FL protein levels and a concomitant increase of TrkB-ICD protein levels, when compared to MCI patients without AD diagnosis. Although only preliminary (Fig. 8), this data is an important starting point to unveil whether these proteins could be applicable in the biomarker field.

### TrkB-ICD as a key player in AD pathophysiology

The inventors demonstrated that TrkB-ICD is really affecting the physiological environment, through its overexpression in primary neurons and wild-type animals. Using lentiviral transduction of primary neurons, the inventors demonstrated that TrkB-ICD promoted transcriptomic alterations, being the up-regulated genes mainly associated with the cellular compartment, signal transduction pathways and Alzheimer's disease. It should be highlighted that TrkB-ICD-induced transcriptomic alterations are present in 20 of 21 chromosomes (Fig. 9).

Through intra-hippocampal injection of lentivirus in wild-type animals, the inventors observed that TrkB-ICD-expressing animals presented memory impairment (Novel-object recognition test), without alterations in locomotor and anxious parameters (Fig. 10). After behavioral analysis, animals were functionally evaluated by electrophysiological assays. The inventors observed no alterations in paired pulse facilitation and post-tethanic pulse in animals tested. However, long-term potentiation, which is the neurophysiological basis of memory processes, since it measures synaptic plasticity, was increased in TrkB-ICD-expressing animals, when compared to both control groups (Fig. 11).

Briefly, the inventors observed that the TrkB-ICD fragment *per se* plays a role in normal physiology, by alterations at genomic, functional and behavioral levels. Actually, this fragment alone promoted a dysfunctional synaptic plasticity and memory impairments.

### Methods

Methods used are detailed in parent application EP'999 and are herein incorporated by reference.

### Wester-Blot, qPCR and Mass Spectrometry for Human Samples

Western-Blot, mRNA and MS analysis were performed in a subset of the samples; demographics are described in Supplementary Table 1 as disclosed in parent application EP'999 which is herein incorporated by reference.

### Western Blot analysis

After treatments, as indicated in figure descriptions, primary neuronal cultures (at DIV 8 or DIV14-16) and rat cortical samples were washed with ice-cold PBS and lysed as described above. All samples were prepared with the same amount of total protein (40 µg), unless stated otherwise. A loading buffer (350 mM Tris pH 6.8, 10% SDS, 30% glycerol, 600 mM Dithiothreitol, 0.06% bromophenol blue) was added and the mixture was boiled at 95-100°C for 10 min. Next, all samples and the molecular weight size marker (NZYTech, Lisbon, Portugal) were loaded and separated on 10% SDS-PAGE in a standard migration buffer (25 mM Tris pH 8.3, 192 mM Glycine, 10% SDS), at a constant voltage between 80-120 mV. Then, proteins were transferred onto PVDF membranes (GE Healthcare, Buckinghamshire, UK), previously soaked for 5 min in a standard buffer (25 mM Tris pH = 8.3, 192 mM Glycine, 15% methanol) for wet transfer conditions. After 1h30 of transfer, membranes were stained with Ponceau S solution (#09276-6X1EA-F, Sigma-Aldrich, St. Louis, MO, USA) to evaluate protein transfer efficacy. Membranes were blocked with a 3% (wt/vl) Bovin Serum Albumin in Tris-buffered saline (20 mM Tris base, 137 mM NaCl and 0.1% Tween-20) and subsequently incubated overnight with a primary antibody, the Trk-FL rabbit polyclonal antibody (1:1000) raised against the C-terminus (C-14) (#sc-11, Santa Cruz Biotechnology, Dallas, TX, USA) and for 1h at RT with goat anti-rabbit IgG-horseradish peroxidase-conjugated secondary antibody (1:10000) (#sc-2004, Santa Cruz Biotechnology, Dallas, TX, USA). Finally, immunoreactivity was detected using the ECL system (ECL chemiluminescence detection system, GE Healthcare), an appropriate exposure time in a ChemiDoc (Bio-Rad, XRS⁺) and quantified by the digital densitometry tool of ImageJ 1.45 software (Bethesda, MD, USA). GAPDH or β-actin were used as loading control (mouse anti-GAPDH (1:5000), #AM4300, Thermo Fischer Scientific, Waltham, MA, USA; mouse anti-β-actin (1:1000), #ab8226, Abcam Biochemicals (Cambridge, MA, USA); goat anti-mouse IgG-horseradish peroxidase-conjugated secondary antibody (1:10000), #sc-2005, Santa Cruz Biotechnology, Dallas, TX, USA). Figure 13 shows all the samples quantified and uncropped gels and blots with molecular weight standards; two samples were not considered for the analysis (one due to low quality; the other was judged as a statistical outlier, with a quantification value greater than the mean + 2.5 s.d. of the remaining observations).

### TAT-TrkB Peptide Design and Structural Prediction

The final TAT-TrkB peptide (Fig. 1d) contains a linker (KK) connecting (1) the TAT protein transduction domain motif derived from the HIV-1 protein Trans-Activator of Transcription (TAT) (YGRKKRRQRRR) and (2) the TrkB-FL sequence (515-524) that is cleaved by the amyloid-β peptide.

Several TAT-TrkB peptide sequences were simulated: final TAT-TrkB - YGRKKRRQRRRKKFGITNSQLKP; first version without the linker: YGRKKRRQRRRPQYFGITNSQLKPDTF; with PP as linker: YGRKKRRQRRRPPFGITNSQLKP; with Q as linker: YGRKKRRQRRRQFGITNSQLKP; with E as linker: YGRKKRRQRRREFGITNSQLKP (Fig. 5). For BEB assays, 6-FAM fluorophore was fused to the N-terminal of the final version of TAT-TrkB peptide.The peptides were synthesized by GenScript Corporation (Piscataway, NJ, USA) and prepared in sterile MiliQ water as 1 mM stock solution.

For structural predictions, ConSSert software (available at http://ares.tamu.edu/conSSert/)²¹ and the SSPro8 tool from Scratch server (available at http://scratch.proteomics.ics.uci.edu/)²² were used. For 3D structural predictions, the PEP-FOLD 3.0 software via the online server Mobyle@RPBS v1.5.1 (http://mobyle.rpbs.univ-paris-diderot.fr/)²³ was used. After initial studies aiming to determine peptide concentration for optimal activity, all the remaining experiments were performed using TAT-TrkB 20µM, which is within the concentration range of similar peptides previously described^{8,13,24}.

### Calpain in vitro Assays and Rat Brain Homogenate Preparation

For calpain enzymatic assays, rat cortical tissue or neuronal cultured cells were homogenized in Radio Immuno Precipitation Assay (RIPA) buffer, containing 50 mM Tris-HCl [pH = 7.5], 150 mM NaCl, 5 mM ethylenediamine tetra-acetic acid, 0.1% sodium dodecyl sulfate (SDS), 1% [vl/vl] Triton X-100, protease inhibitors (Roche, Penzberg, Germany) and phosphatase inhibitors (10 mM NaF and 5 mM Na₃VO₄). For tissue homogenization, Vibracell VC250 Ultrasonic Liquid Processor was used (Sonics & Materials, Inc, CT, USA). Protein concentration was measured using Bio-Rad DC reagent (Bio-Rad Laboratories, Berkeley, CA, USA) after homogenates clarification by centrifugation (16,000g, 4ºC, 10 min). The purified rat m-calpain (1 or 2 enzymatic units, EU) (Calbiochem, San Diego, CA, USA) was incubated for 30 min at 25 ºC in a 100 µL final volume of lysis buffer containing the homogenate protein (100 µg for rat brain cortex experiments and 65µg for neuronal cultured cells), 2 mM of CaCl₂ and TAT-TrkB peptide (5, 20 or 50µM). Enzymatic digestion was stopped by boiling the samples at 95º C in the presence of the denaturing SDS-sample buffer, proceeding immediately to Western Blot analysis.

### Blood-Endothelial Barrier (BEB) Translocation and Integrity Assays

To evaluate the ability of the TAT-TrkB peptide to translocate the BEB, the inventors used a well-established *in vitro* model ¹⁵. It comprises a transwell system with an insert in which bEnd.3 brain endothelioma cells (ATTCC-CRL-2299, Lot. 59618606) are cultured for 9 days. In this model, the apical chamber replicates the blood compartment, while the basolateral chamber replicates the brain compartment (Fig. 7a). For these experiments, 6-FAM was fused to the N-terminal of the TAT-TrkB peptide, in order to detect the peptide by fluorescence. The TAT-TrkB-6-FAM peptide (5 µM, diluted in DMEM without phenol red) was delivered to the apical compartment. After incubation times of 1h, 4h and 24h, in different inserts, medium from the apical and basolateral compartments was collected and fluorescence measured at 495 nm excitation and 517 nm emission wavelengths using the Varioskan LUX Multimode microplate reader (Thermo Scientific, Rockford, IL, USA ThermoFisher Scientific). Peptide translocation was quantified as the fluorescence detected in the basolateral compartment normalized to the sum of the fluorescence detected in both compartments. Immediately after the TAT-TrkB.6-FAM translocation assays, *in vitro* integrity studies were performed. For these, FD4 fluorescent probe (diluted in DMEM without phenol red to a final absorbance below 0.1) was added to the apical compartment of the same inserts and incubated for 2h. FD4 is a fluorescently labelled dextran with 4 kDa and negligible translocation when cells integrity is guaranteed. In parallel, FD4 was also loaded to the apical compartment of inserts with untreated cells (negative control), as well as with cells exposed to EGTA (5 mM), together with FD4. EGTA serves as a positive control, since it is known to disrupt tight junctions and other cell-cell adhesions. Again, samples were collected from both compartments and fluorescence measured at 493 nm excitation and 520 nm emission wavelengths using the Varioskan LUX Multimode microplate reader. FD4 basal recovery was quantified as the fluorescence detected in the basolateral compartment normalized to the sum of the fluorescence detected in both compartments. For both assays, values were obtained from triplicates of three independent experiments.

### Acute Hippocampal Slice Preparation

Young adult (7-10 weeks old) male Wistar rats were sacrificed by decapitation under deep isoflurane anaesthesia. The brain was quickly removed, hemisected and both hippocampi were dissected free within ice-cold artificial cerebrospinal fluid (aCSF) (mM: NaCl 124; KCl 3; NaH₂PO₄ 1.25; NaHCOs 26; MgSO₄ 1; CaCl₂ 2; and glucose 10), previously gassed with 95% O₂ and 5% COz, pH 7.4.

Slices (400 µm-thick) were cut perpendicularly to the long axis of the hippocampus with a McIlwain tissue chopper and allowed to recover for at least 60 min in a resting chamber, filled with the gassed aCSF, at room temperature (RT) (22-24ºC).

### MTT Assay

Viability of mature (DIV14-16) primary cortical neuronal cells incubated with vehicle or Aβ species for 24 h was evaluated by the metabolism of 3-(4,5-dimethylthiazol-2-yl)-2,5-diphenyl tetrazolium (MTT) bromide (#CT01, Sigma-Aldrich, MO, USA). MTT was added to the original culture medium and incubated for 3 h. After incubation, the converted die was solubilized with dimethylsulfoxide (DMSO) (Merck, Kenilworth, NJ, USA). Absorbance of converted die was measured at 570 nm with background subtraction at 650 nm in a microplate reader Infinite M200 (Tecan, Männedorf, Switzerland).

### Propidium Iodide (PI) staining and Immunocytochemistry

Immediately after treatments, mature (DIV14-16) primary neuronal cultures were incubated with 500 nM PI (#81845, Sigma-Aldrich, MO, USA) and maintained in an incubator with a humidified atmosphere of 5% COz at 37 ºC for 30 min. Neuronal cultures were then washed with PBS and fixed in 4% paraformaldehyde in PBS (pH = 7.4) for 15 min at RT. Cells were treated with blocking solution (3% [wt/vl] bovine serum albumin, BSA, Sigma-Aldrich, MO, USA) in PBS with 0.1% (vl/vl) Triton X-100 for 1 h to block unspecific binding. After washing, cells were incubated overnight at 4°C with mouse microtubule-associated protein 2 (MAP2) primary antibody (1:200 in blocking solution) (#2603311, Millipore, Billerica, MA, USA), to specifically detect neurons. Afterwards, cells were washed and incubated with goat anti-mouse Alexa Fluor^{®} 568 secondary antibody (1:200 in blocking solution) (#A10042, Invitrogen, Carlsbad, CA, USA), for 1h at RT, in the dark. Coverslips were mounted in Mowiol mounting solution and observed using an inverted fluorescent microscope Axiovert 135 TV (Carl Zeiss Microscopy, Thornwood, NY, USA). Six random microscopic fields per sample of approximately 80 cells were counted and mean values expressed as percentage of PI-positive neurons.

### Spine Density Count

Cells were fixed and treated with blocking solution as described above. Next, cells were incubated with mouse MAP2 primary antibody (1:200 in blocking solution), overnight at 4°C and subsequently washed and incubated with goat anti-mouse Alexa Fluor^{®} 568 secondary antibody (1:200 in blocking solution), for 1h at RT, in the dark. Then, cells were incubated for 30 min with Alexa Fluor^{®} 488 Phalloidin (1:40 in PBS) (#A12379, Invitrogen, Carlsbad, CA, USA), which recognizes filamentous actin (F-actin). F-actin has an important role in the constitution of the cytoskeleton of dendritic spines ³². After washing, coverslips were mounted and cells were imaged as described above. Spine density is expressed as the number of small protrusions (either mushroom-shape spines or filopodia) extending ≤3 µm from their parent dendrite with a distance >25 µm from the cell body, normalized to 10 µm of the parent dendrite, as previously reported ³³. Dendritic spines were counted offline using ImageJ 1.45 (Bethesda, MD, USA). Six neurons per condition per culture were analyzed and in each neuron protrusions were counted in 3 different dendrites.

### mEPSC Recordings

Miniature excitatory postsynaptic currents (mEPSCs) of isolated primary neurons were evaluated by whole-cell patch clamp, immediately after treatments, as routinely in the lab ³⁴. Briefly, cells were visualized with an upright microscope (Axioskop 2FS, Carl Zeiss Microscopy, Thornwood, NY, USA) equipped with differential interference contrast optics using a Zeiss AxioCam MRm camera and a X40 IR-Achroplan objective. The resting membrane potential was measured immediately upon establishing whole-cell configuration. mEPSCs recordings were performed at RT and in voltage-clamp mode (Vh = -60 mV), using an Axopatch 200B amplifier (Molecular Devices, CA, USA). The extracellular media was gassed aCSF supplement with tetrodotoxin (TTX, 500 nM), a sodium channel blocker, and gabazine (GBZ, 50 µM), a GABA_{A} receptor antagonist. Patch pipettes (4- to 7-MΩ resistance) were filled with an internal solution containing (in mM): K-gluconate 125, KCI 11, CaCl₂ 0.1, MgCl₂ 2, EGTA 1, HEPES 10, MgATP 2, NaGTP 0.3, Phosphocreatine 1, pH 7.3, adjusted with 1 M KOH, 280-290 mOsm. Acquired signals were filtered using an in-built, 2-kHz, three-pole Bessel filter, and data were digitized at 5 or 10 kHz under control of the pCLAMP 10 (Molecular Devices, CA, USA) software program. The junction potential was not compensated for, and offset potentials were nulled before giga-seal formation. Small voltage steps (-5 mV, 50 ms) were used to monitor the access resistance throughout experiments. The holding current was also constantly monitored and experiments in which any of these parameters varied by more than 20% were discarded. Analysis of miniature events was performed using the Mini Analysis Software (Synaptosoft Inc., NJ, USA), and event detection threshold was set at three times the root mean square (RMS) baseline (noise). mEPSCs frequency, average amplitude, rise and decay times were established by analysing 5-10 min of recording periods per cell.

### PI and Immunohistochemistry

After treatments, hippocampal slices were incubated with 500 nM PI for 30 min at RT in gassed aCSF and then fixed in 4% paraformaldehyde in PBS (pH = 7.4) for 15 min at RT. Next, slices were dehydrated with sequential solutions of 10%, 20% and 30% sucrose, embedded in Tissue-Tek O.C.T. (Sakura Finetek, Torrence, CA, USA) and stored at -80°C. Serial 20-µm-thick hippocampal sections were cut on a cryostat (Leica Biosystems, Wetzlar, Germany) and mounted on SuperFrost-Plus glass slides (Thermo Scientific, Rockford, IL, USA). Hippocampal slices were then rehydrated and boiled three times in 10 mM citrate buffer, pH 6. Next, slices were treated with blocking solution (3% [wt/vl] BSA) in PBS with 0.1% (vl/vl) Triton X-100 for 1 h. After washing, slices were incubated with mouse MAP2 primary antibody (1:200 in blocking solution), overnight at 4°C. Afterwards, slices were washed and incubated with goat anti-mouse Alexa Fluor^{®} 568 secondary antibody (1:200 in blocking solution), for 1h at RT, in the dark. After incubation with 5 µg/mL Hoechst dye (#33258, Sigma-Aldrich, St. Louis, MO, USA) for 5 min, slices were washed, mounted in Mowiol mounting solution and sections were imaged with an inverted fluorescent microscope Axiovert 135 TV (Carl Zeiss Microscopy, Thornwood, NY, USA). CA1 and CA3 areas were analyzed and mean values are expressed as percentage of PI-positive cells and percentage of PI-positive neurons.

### Extracellular Ex Vivo Recordings

Slices were transferred to a submerging chamber (1 ml) and continuously superfused at a 3 ml/min rate with gassed aCSF at 32ºC. Perfusion tubes were coated with 0.1 mg/ml BSA prior to experiments to avoid adsorption of BDNF to the tubes ³⁵. Evoked field excitatory postsynaptic potentials (fEPSP) were recorded extracellularly through a microelectrode filled with 4 M NaCl (2-8 MQ resistance) placed in the *stratum radiatum* of the CA1 area, as previously described ^{34,35}. One pathway of the Schaffer collateral/commissural fibers was stimulated (rectangular pulses of 0.1 ms duration) at every 15 s (for I/O curves, basal synaptic transmission and Paired-Pulse Facilitation (PPF) recordings) or every 20 s (for LTP recordings), by a bipolar concentric wire electrode placed on the Schaffer fibers in the stratum *radiatum,* in the CA1 area. The initial intensity of the stimulus was adjusted to obtain a submaximal fEPSP slope with a minimum population spike contamination, near one-fifth of the fEPSP slope obtained with supramaximal stimulation. The averages of eight consecutive fEPSPs were obtained and the slope of the initial phase of the potential was quantified. Recordings were obtained with an Axoclamp 2B amplifier (Axon Instruments, Foster City, CA), digitized and continuously stored on a personal computer with the LTP program ³⁶. All the protocols detailed below were started only after a stable baseline of at least 20 minutes.

*Input*/*Output Curves.* The stimulus delivered to the slice was decreased until no fEPSP was evoked and subsequently increased by steps of 20 µA. Data from three consecutive averaged fEPSPs were collected for each stimulation intensity. The range of inputs delivered to slices was typically from 80 µA to a supramaximal stimulation of 320 µA. Maximum slope values were obtained by extrapolation upon nonlinear fitting of the I/O curve. Whenever the stimulus was intense enough to elicit a population spike (depolarization that follows the fEPSP), its amplitude was measured. Each popspike amplitude replicate was plotted as a function of the slope of the associated fEPSP and the popspike/fEPSP ratio calculated as the quotient between these values ³⁴.

*Long-Term Potentiation induction and saturation.* A weak LTP-inducing θ-burst protocol consisting of four trains of 100 Hz, four stimuli, separated by 200 ms was used (4x4) ^{34,37}. The intensity of the stimulus was never changed. LTP magnitude was quantified as the normalized change in the average slope of the fEPSP taken from 50 to 60 min after LTP induction in relation to the average slope of the fEPSP measured during the 10 min that preceded the induction of LTP.

For the experiments in **Figure 2****,** Brain-Derived Neurotrophic Factor (BDNF, 30 ng/ml) was added to the superfusing solution 20 min before the induction of LTP. Controls for these experiments were performed in different slices, without BDNF superfusion, on the same day. Generally, when testing BDNF effect on synaptic plasticity, two independent pathways of the same hippocampal slice are used, one serving as test and the other as internal control ^{35,38}. However, because in the present experimental design slices were previously exposed to vehicle/Aβ for 3 h, we chose to test BDNF effect in different slices from the same rats. Otherwise, time between the end of vehicle/Aβ treatment and LTP induction would be consistently longer when BDNF was to be tested (as BDNF can only be added to the superfusing solution after LTP is induced in the first pathway).

For the experiments in **Figure 3a****-d,** in order to test LTP saturation, we induced three consecutive θ-burst (4x4) paradigms in the same pathway spaced by 1 h intervals. We based our experimental design on previous results showing that LTP has a refractory period of 60 min before which a new LTP cannot be effectively induced ¹⁶. We calculated the Saturation Index (SI) as the quotient of the sum of the three LTP magnitudes by the magnitude of the first LTP [(LTP1+LTP2+LTP3)/LTP1) (hence, if SI ≈ 3, LTP is fully saturated; if SI > 3, LTP is not saturated; the higher the SI, the more desaturated LTP is).

For the experiments in **Figure 3e****-f,** BDNF's scavenger, TrkB-Fc (2 µg/ml), was added to the superfusion solution 30 min before induction of LTP and remained in the bath up to the end of the experiment.

*Paired-Pulse Facilitation (PPF) Recordings.* PPF was quantified as the ratio between the second versus the first fEPSP slope (fEPSP2/fEPSP1) responses at different interstimulus intervals (10-150 ms) ³⁴.

### [³H]Glutamate Release Assays

*Isolation of Synaptosomes.* The synaptosomal fraction from rat hippocampi was prepared as routinely in the lab ³⁹. Hippocampal slices (male Wistar rats, 7-10 weeks old) were prepared and treated with vehicle or Aβ₁₋₄₂, as described above. Then, slices were added to 5 ml of ice-cold isosmotic sucrose solution (0.32 M, containing 1 mM EDTA, 1 mg/ml BSA, 10 mM HEPES, pH 7.4), and homogenized in a Potter-Elvehjem homogenizer with a Teflon piston (four up-and-down strokes) and the volume completed to 10 ml with the sucrose solution. After a first centrifugation at 3,000g for 10 min (Heraeus sepatech - Biofuge 28RS centrifuge with 3746 rotor), the supernatant was collected and centrifuged at 14,000g for 12 min. The whole procedure was conducted at 4°C. The pellet was resuspended in 1 ml 45% Percoll in KHR solution consisting of (in mM) NaCl 140, EDTA 1, HEPES 10, KCl 5, and glucose 5, and centrifuged at 16,000g for 2 min. The synaptosomal fraction corresponds to the top buoyant layer and was collected from the tube. Percoll was removed by two washes with 1 ml KHR solution centrifuged at 16,000g for 2 min each; synaptosomes were then kept on ice and used within 3 hours. The synaptosomal protein concentration was assayed using the Bio-Rad DC^{™} protein assay and BSA as standard.

*[³H]Glutamate Release from Hippocampal Synaptosomes.* [³H]Glutamate release from hippocampal synaptosomes was performed as routinely in the lab ³⁹. Synaptosomes (protein concentration 1-2 mg/ml) were resuspended in oxygenated Krebs medium (in mM: NaCl 125, KCI 3, NaH2PO4 1, glucose 10 NaHCO3 25, CaCl2 1.5 and MgSO4 1.2) and allowed to equilibrate for 5 min at 37°C. From this time onward, all solutions applied to the synaptosomes were kept at 37ºC and continuously gassed with O₂/CO₂ (95%/5%). Then, synaptosomes were loaded with 0.2 µM [³H]glutamate (specific activity 30-60 Ci/mmol) for 5 min and equally layered over Whatman GF/C filters (Milipore, MA, USA) onto an eight-chamber superfusion apparatus (flow rate, 0.6 ml/min; chamber volume, 90 µl).The constant and rapid flow rate washes out endogenously related substances, thus ensuring drug effect specificity ⁴⁰. After a 20 min washout period, the effluent was collected for 40 min in 2 min intervals. Glutamate release from synaptosomes was elicited during 2 min with a high-K⁺ solution (15 mM, isomolar substitution of Na⁺ with K⁺ in the perfusion buffer) at the 5th [first stimulation period (S1)] and 29th [second stimulation period (S2)] min after starting sample collection. BDNF (30 ng/ml) was added to the superfusion medium at the 9th min, therefore before S2, and remained in the bath up to the end of the experiments (as indicated by the horizontal bars in Fig. 2b-e). Control curves, in the absence of BDNF, were performed in parallel with the same synaptosomal batch.

*Calculation of drug effects on glutamate release.* At the end of each experiment, aliquots (500 µl) of each sample as well as the filters from each superfusion chamber were analyzed by liquid scintillation counting. The fractional release was expressed as the percentage of total radioactivity present in the synaptosomes at each time point. The amount of radioactivity released by each pulse of K⁺ (S1 and S2) was calculated by integration of the area of the peak upon subtraction of the estimated basal tritium release. Each condition was tested in duplicate. Hence, in each experiment, two synaptosome-loaded chambers were used as control chambers, the others being used as test chambers. Each one of the chambers was used as control or as one of the test conditions in different days.

### Drugs and Reagents

Aβ₂₅₋₃₅ was purchased from Bachem (Bubendorf, Switzerland) and prepared in 1 mg/ml stock solution in sterile MiliQ water. Aβ₁₋₄₂ (1 mg/ml) (A-42-T, GenicBio, Shanghai, China) was suspended in phosphate-buffered saline (PBS) (supplemented with 0.1% ammonia solution) and adjusted to a final pH 7.2. BDNF was generously supplied by Regeneron Pharmaceuticals (Tarrytown, NY) in a 1.0 mg/ml stock solution in 150 mM NaCl, 10 mM sodium phosphate buffer, and 0.004% Tween 20. The recombinant human TrkB/Fc chimera, TrkB-Fc, was purchased from R&D systems (Minneapolis, MN) and made up in a 50 µg/ml stock solution in PBS with 0.1% BSA. TTX and gabazine were purchased from Abcam Biochemicals (Cambridge, MA, USA) and prepared in distilled water as 1 and 10 mM stock solutions, respectively. MDL28170 was bought from Tocris (Ballwin, MO, USA), and prepared in a 20 mM stock solution in DMSO. [3H]Glutamate (L-[G-3H]glutamic acid, specific activity 45 Ci/mmol) was purchased from PerkinElmer Life Sciences (Waltham, MA, USA). Percoll, EDTA, KCI, MgSO4·7H2 O, NaCl, and NaH2PO4·H2O were bought from Sigma-Aldrich (St. Louis, MO, USA); glucose, sucrose, HEPES, NaH2PO4 ·H2O, NaOH, and HCl were bought from Merck (Kenilworth, NJ, USA). The other reagents, drugs and antibodies needed for this work were purchased and prepared as described in the respective sections above.

### Statistical Analysis

Sample sizes were determined based on preliminary results or similar experiments carried out in the past. Values are presented individually in scattered dot blots, with bars overlaid to depict mean±s.e.m or mean±95%CI, as indicated. Hyperbolic regressions (Fig. 1g) and linear regressions were fitted using the least squares method. Statistical analyses were designed under the assumption of normal distribution and similar variance among groups. For inference analysis, paired t-tests, unpaired t-tests or one-way ANOVA followed by a Bonferroni's multiple comparison post hoc tests were used, as specified in the figure legends and detailed in Supplementary Table 2. P < 0.05 was considered statistically significant. All statistical analysis was performed on Prism 8 (version 8.1.2, GraphPad Software Inc.).

Any of the aspects and embodiments described herein can be combined with any other aspect or embodiment as disclosed in the Summary of the Invention, in the Drawings, or in the Detailed Description of the Invention, including the below specific, non-limiting, examples/embodiments of the present invention

The more general and advantageous configurations of the present invention are described in the Summary of the invention. Such configurations are detailed below in accordance with other advantageous and/or preferred embodiments of implementation of the present invention.

As will be clear to one skilled in the art, the present invention should not be limited to the embodiments described herein, and a number of changes are possible which remain within the scope of the present invention.

The disclosure should not be seen in any way restricted to the embodiments described and a person with ordinary skill in the art will foresee many possibilities to modifications thereof.

The above-described embodiments are combinable.

The following claims further set out particular embodiments of the disclosure.

### References:

1. Huang, E. J. & Reichardt, L. F. Neurotrophins: roles in neuronal development and function. Annu. Rev. Neurosci. 24, 677-736 (2001).
2. Connor, B. et al. Brain-derived neurotrophic factor is reduced in Alzheimer's disease. Brain Res. Mol. Brain Res. 49, 71-81 (1997).
3. Ferrer, I. et al. BDNF and full-length and truncated TrkB expression in Alzheimer disease. Implications in therapeutic strategies. J. Neuropathol. Exp. Neurol. 58, 729-39 (1999).
4. Phillips, H. S. et al. BDNF mRNA is decreased in the hippocampus of individuals with Alzheimer's disease. Neuron 7, 695-702 (1991).
5. Tanqueiro, S. R. et al. Inhibition of NMDA Receptors Prevents the Loss of BDNF Function Induced by Amyloid-Beta. Front. Pharmacol. (2018).
6. Jerónimo-Santos, A. et al. Dysregulation of TrkB Receptors and BDNF Function by Amyloid-β Peptide is Mediated by Calpain. Cereb. Cortex 25(9), 3107-21 (2015).
7. Fonseca-Gomes, J. et al. TrkB-ICD Fragment, Originating From BDNF Receptor Cleavage, Is Translocated to Cell Nucleus and Phosphorylates Nuclear and Axonal Proteins. Front. Mol. Neurosci. (2019). doi:10.3389/fnmol.2019.00004
8. Tejeda, G. S., Esteban-Ortega, G. M., San Antonio, E., Vidaurre, Ó. G. & Díaz-Guerra, M. Prevention of excitotoxicity-induced processing of BDNF receptor TrkB-FL leads to stroke neuroprotection. EMBO Mol. Med. e9950 (2019). doi:10.15252/emmm.201809950
9. Wadia, J. S. & Dowdy, S. F. Transmembrane delivery of protein and peptide drugs by TAT-mediated transduction in the treatment of cancer. Adv. Drug Deliv. Rev. 57, 579-96 (2005).
10. Schwarze, S. R., Ho, A., Vocero-Akbani, A. & Dowdy, S. F. In vivo protein transduction: delivery of a biologically active protein into the mouse. Science 285, 1569-72 (1999).
11. Azzarito, V., Long, K., Murphy, N. S. & Wilson, A. J. Inhibition of α-helix-mediated protein-protein interactions using designed molecules. Nat. Chem. 5, 161-73 (2013).
12. Rao, T. et al. Truncated and Helix-Constrained Peptides with High Affinity and Specificity for the cFos Coiled-Coil of AP-1. PLoS One 8, e59415 (2013).
13. Li, S. et al. Swedish mutant APP-based BACE1 binding site peptide reduces APP β-cleavage and cerebral Aβ levels in Alzheimer's mice. Sci. Rep. 5, 11322 (2015).
14. Chen, X., Zaro, J. L. & Shen, W.-C. Fusion protein linkers: property, design and functionality. Adv. Drug Deliv. Rev. 65, 1357-69 (2013).
15. Neves, V. et al. Novel Peptides Derived from Dengue Virus Capsid Protein Translocate Reversibly the Blood-Brain Barrier through a Receptor-Free Mechanism. ACS Chem. Biol. 12, 1257-1268 (2017).
16. Kramar, E. A. et al. Synaptic evidence for the efficacy of spaced learning. Proc. Natl. Acad. Sci. (2012). doi:10.1073/pnas.1120700109
17. Diógenes, M. J. et al. Enhancement of LTP in aged rats is dependent on endogenous BDNF. Neuropsychopharmacology 36, 1823-36 (2011).
18. Goll, D. E., Thompson, V. F., Li, H., Wei, W. & Cong, J. The calpain system. Physiol. Rev. 83, 731-801 (2003).
19. Marttinen, M. et al. A multiomic approach to characterize the temporal sequence in Alzheimer's disease-related pathology. Neurobiol. Dis. 124, 454-468 (2019).
20. Braak, H., Alafuzoff, I., Arzberger, T, Kretzschmar, H. & Del Tredici, K. Staging of Alzheimer disease-associated neurofibrillary pathology using paraffin sections and immunocytochemistry. Acta Neuropathol. 112, 389-404 (2006).
21. Kieslich, C. A., Smadbeck, J., Khoury, G. A. & Floudas, C. A. conSSert: Consensus SVM Model for Accurate Prediction of Ordered Secondary Structure. J. Chem. Inf. Model. 56, 455-61 (2016).
22. Pollastri, G., Przybylski, D., Post, B. & Baldi, P. Improving the prediction of protein secondary structure in three and eight classes using recurrent neural networks and profiles. Proteins 47, 228-35 (2002).
23. Lamiable, A. et al. PEP-FoLD3: faster de novo structure prediction for linear peptides in solution and in complex. Nucleic Acids Res. 44, W449-W454 (2016).
24. Gamir-Morralla, A. et al. Development of a neuroprotective peptide that preserves survival pathways by preventing Kidins220/ ARMS calpain processing induced by excitotoxicity. Cell Death Dis. 6, (2015).
25. Giuffrida, M. L. et al. Beta-Amyloid Monomers Are Neuroprotective. J. Neurosci. 29, 10582-10587 (2009).
26. Vicente Miranda, H. et al. Glycation potentiates α-synuclein-associated neurodegeneration in synucleinopathies. Brain (2017). doi:10.1093/brain/awx056
27. Schiff, S. J. & Somjen, G. G. The effects of temperature on synaptic transmission in hippocampal tissue slices. Brain Res. 345, 279-84 (1985).
28. Chen, Q. S., Kagan, B. L., Hirakura, Y. & Xie, C. W. Impairment of hippocampal long-term potentiation by Alzheimer amyloid beta-peptides. J. Neurosci. Res. 60, 65-72 (2000).
29. Ripoli, C. et al. Effects of different amyloid β-protein analogues on synaptic function. Neurobiol. Aging (2013). doi:10.1016/j.neurobiolaging.2012.06.027
30. Dahlgren, K. N. et al. Oligomeric and fibrillar species of amyioid-β peptides differentially affect neuronal viability. J. Biol. Chem. 277, 32046-32053 (2002).
31. Karran, E., Mercken, M. & Strooper, B. De. The amyloid cascade hypothesis for Alzheimer's disease: An appraisal for the development of therapeutics. Nature Reviews Drug Discovery (2011). doi:10.1038/nrd3505
32. Koskinen, M. & Hotulainen, P. Measuring F-actin properties in dendritic spines. Front. Neuroanat. 8, 74 (2014).
33. Alonso, M., Medina, J. H. & Pozzo-Miller, L. ERK1/2 Activation Is Necessary for BDNF to Increase Dendritic Spine Density in Hippocampal CA1 Pyramidal Neurons. Learn. Mem. 11, 172-178 (2004).
34. Dias, R. B. et al. Erythropoietin induces homeostatic plasticity at hippocampal synapses. Cereb. Cortex (2018). doi:10.1093/cercor/bhx159
35. Rodrigues, T. M., Jerónimo-Santos, A., Sebastião, A. M. & Diógenes, M. J. Adenosine A2A Receptors as novel upstream regulators of BDNF-mediated attenuation of hippocampal Long-Term Depression (LTD). Neuropharmacology (2014). doi:10.1016/j.neuropharm.2013.12.010
36. Anderson, W. W. & Collingridge, G. L. The LTP Program: a data acquisition program for on-line analysis of long-term potentiation and other synaptic events. J. Neurosci. Methods 108, 71-83 (2001).
37. Pinho, J. et al. Enhanced LTP in aged rats: Detrimental or compensatory? Neuropharmacology (2017). doi:10.1016/j.neuropharm.2016.11.017
38. Fontinha, B. M., Diógenes, M. J., Ribeiro, J. A. & Sebastião, A. M. Enhancement of long-term potentiation by brain-derived neurotrophic factor requires adenosine A2A receptor activation by endogenous adenosine. Neuropharmacology (2008). doi:10.1016/j.neuropharm.2008.01.011
39. Vaz, S. H., Lérias, S. R., Parreira, S., Diógenes, M. J. & Sebastião, A. M. Adenosine A2A receptor activation is determinant for BDNF actions upon GABA and glutamate release from rat hippocampal synaptosomes. Purinergic Signal. (2015). doi:10.1007/s11302-015-9476-1
40. Raiteri, L. & Raiteri, M. Synaptosomes still viable after 25 years of superfusion. Neurochem Res (2000). doi:10.1023/A:1007648229795

## Claims

1. Use of TrkB-FL, TrkB-ICD, TrkB-T', the TrkB-T':TrkB-FL ratio, or the TrkB-ICD:TrkB-FL ratio in the *in vitro* diagnosis of Alzheimer Disease (AD) or for determining the stage of AD.

2. A method for diagnosing AD or for determining the stage of AD, comprising:
i) comparing the amount of TrkB-FL and/or TrkB-ICD measured in a sample obtained from a subject to a reference amount, and;
ii)
a) determining that said subject has AD, or determining that said subject has a more advanced stage of AD than that represented by the reference amount, if said amount of TrkB-FL is lower than said reference amount and/or if said amount of TrkB-ICD is higher than said reference amount; or
b) determining that said subject has the same stage of AD or AD diagnosis as that represented by the reference amount if said amount of TrkB-FL and/or TrkB-ICD is the same or similar to said reference amount; or
c) determining that said subject does not have AD, or determining that said subject has a less advanced stage of AD than that represented by the reference amount, if said amount of TrkB-FL is higher than said reference amount and/or if said amount of TrkB-ICD is lower than said reference amount;
said method optionally comprising prior to step i), measuring the amount of TrkB-FL and/or TrkB-ICD in a sample obtained from a subject.

3. A method for diagnosing AD or for determining the stage of AD, comprising:
i) comparing the amount of TrkB-T' measured in a sample obtained from a subject to a reference amount, and
ii) a) determining that said subject has AD, or determining that said subject has a more advanced stage of AD than that represented by the reference amount, if said amount of TrkB-T' is higher than said reference amount; or b) determining that said subject has the same stage of AD or AD diagnosis as that represented by the reference amount if said amount of TrkB-T' is the same or similar to said reference amount; or c) determining that said subject does not have AD, or determining that said subject has a less advanced stage of AD than that represented by the reference amount, if said amount of TrkB-T' is lower than said reference amount;
said method optionally comprising prior to step i), measuring the amount of TrkB-T' in a sample obtained from a subject.

4. The method of any one of claims 2 to 3, wherein the reference amount is representative of the amount of TrkB-T', TrkB-FL, and/or TrkB-ICD in a subject without AD, in a subject with mild AD, in a subject with moderate AD, in a subject with severe AD, determined from said subject before disease onset, determined from said subject after diagnosis of mild AD, determined from said subject after diagnosis with moderate AD, determined from said subject after diagnosis with severe AD, or determined from said subject before treatment.

5. A method for diagnosing AD or for determining the stage of AD, comprising:
i) comparing the TrkB-ICD:TrkB-FL ratio measured in a sample obtained from a subject to a reference value, and
ii) a) determining that said subject has AD or determining that said subject has a more advanced stage of AD than that represented by the reference value, if said TrkB-ICD:TrkB-FL ratio is higher than said reference value; or b) determining that said subject has the same stage of AD or AD diagnosis as that represented by the reference value if said TrkB-ICD:TrkB-FL ratio is the same or similar to said reference value; or c) determining that said subject does not have AD, or determining that the subject has a less advanced stage of AD than that represented by the reference value, if said TrkB-ICD:TrkB-FL ratio is lower than said reference value;
wherein said method comprises prior to step i), measuring the amount of TrkB-FL and TrkB-ICD in a sample obtained from the subject, and calculating the TrkB-ICD:TrkB-FL ratio in said sample.

6. A method for diagnosing AD or for determining the stage of AD, comprising:
i) comparing the TrkB-T':TrkB-FL ratio measured in a sample obtained from a subject to a reference value, and
ii) a) determining that said subject has AD or determining that said subject has a more advanced stage of AD than that represented by the reference value, if said TrkB-T':TrkB-FL ratio is higher than said reference value; or b) determining that said subject has the same stage of AD or AD diagnosis as that represented by the reference value if said TrkB-T':TrkB-FL ratio is the same or similar to said reference value; or c) determining that said subject does not have AD, or determining that the subject has a less advanced stage of AD than that represented by the reference value, if said TrkB-T':TrkB-FL ratio is lower than said reference value;
wherein said method comprises prior to step i), measuring the amount of TrkB-FL and TrkB-T' in a sample obtained from the subject, and calculating the TrkB-T':TrkB-FL ratio in said sample.

7. The method of any one of claims 5 to 6, wherein the reference value is representative of the TrkB-ICD:TrkB-FL ratio or the TrkB-T':TrkB-FL ratio in a subject without AD, in a subject with mild AD, in a subject with moderate AD, in a subject with severe AD, determined from said subject before disease onset, determined from said subject after diagnosis of mild AD, determined from said subject after diagnosis with moderate AD, determined from said subject after diagnosis with severe AD, or determined from said subject before treatment.

8. The method of any one of claims 5 to 7, wherein said subject is diagnosed with AD if the subject's measured TrkB-ICD:TrkB-FL ratio or TrkB-T':TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to a reference value representative of a subject without AD.

9. A method for diagnosing AD, comprising:
i) comparing the TrkB-ICD:TrkB-FL ratio or the TrkB-T':TrkB-FL ratio measured in a sample obtained from a first subject with mild cognitive impairment to a reference value obtained from at least one subject without AD, and
ii) a) determining that the first subject has AD if said TrkB-ICD:TrkB-FL ratio or TrkB-T':TrkB-FL ratio is increased by at least 5%, at least 10%, at least 20%, at least 30%, at least 40%, at least 50%, at least 60%, at least 70%, at least 80%, at least 90%, at least 100%, at least 150%, at least 200%, or at least 300% in comparison to said reference value; or b) determining that the first subject does not have AD if said TrkB-ICD:TrkB-FL ratio or TrkB-T':TrkB-FL ratio is not increased compared to said reference value;
wherein said method optionally further comprises prior to step i): measuring the amount of TrkB-FL and TrkB-ICD in a sample obtained from the first subject with mild cognitive impairment, and calculating the TrkB-ICD:TrkB-FL ratio; and/or, measuring the amount of TrkB-FL and TrkB-T' in a sample obtained from the first subject with mild cognitive impairment, and calculating the TrkB-T':TrkB-FL ratio.

10. A method for monitoring the efficacy of an AD treatment with an agent, comprising:
i) calculating an expected amount of TrkB-T', TrkB-FL, and/or TrkB-ICD based upon an amount of TrkB-T', TrkB-FL, and/or TrkB-ICD measured in a first sample obtained from a subject with AD and the time elapsed since the first sample was obtained; and;
ii) comparing: an amount of TrkB-T', TrkB-FL, and/orTrkB-ICD measured in a second sample obtained from the subject after administration of an agent to the subject, to said expected amount of TrkB-T', TrkB-FL, and/or TrkB-ICD; and
iii) a) determining that the treatment is efficacious if the amount of TrkB-FL in the second sample is higher than the expected amount ofTrkB-FL, if the amount ofTrkB-ICB in the second sample is lower than the expected amount ofTrkB-ICB, and/or if the amount of TrkB-T' in the second sample is lower than the expected amount of TrkB-T'; or b) determining that the treatment is not efficacious if the amount of TrkB-FL in the second sample is lower than or the same as the expected amount of TrkB-FL, if the amount ofTrkB-ICB in the second sample is higher than orthe same as the expected amount of TrkB-ICB, and/or if the amount of TrkB-T' in the second sample is higher than or the same as the expected amount of TrkB-T';
wherein said method optionally further comprising, prior to step i), measuring the amount of TrkB-T', TrkB-FL, and/or TrkB-ICD in a first sample obtained from a subject, and prior to step ii), measuring the amount of TrkB-T', TrkB-FL, and TrkB-ICD in a second sample obtained from said subject after administration of an agent to the subject.

11. A method for monitoring the efficacy of an AD treatment with an agent, comprising:
i) calculating an expected TrkB-ICD:TrkB-FL ratio orTrkB-T':TrkB-FL ratio based upon a TrkB-ICD:TrkB-FL ratio or TrkB-T':TrkB-FL ratio measured in a first sample obtained from a subject with AD and the time elapsed since the first sample was obtained; and
ii) comparing: a TrkB-ICD:TrkB-FL ratio or TrkB-T':TrkB-FL ratio measured in a second sample obtained from the subject after administration of an agent to the subject, to said expected TrkB-ICD:TrkB-FL ratio or TrkB-T':TrkB-FL ratio; and
iii) a) determining that the treatment is efficacious if the TrkB-ICD:TrkB-FL ratio in the second sample is lower than the expected TrkB-ICD:TrkB-FL ratio, or determining that the treatment is efficacious if the TrkB-T':TrkB-FL ratio in the second sample is lower than the expected TrkB-T':TrkB-FL ratio; or b) determining that the treatment is not efficacious if the TrkB-ICD:TrkB-FL ratio in the second sample is higher than or the same as the expected TrkB-ICD:TrkB-FL ratio, or determining that the treatment is not efficacious if the TrkB-T':TrkB-FL ratio in the second sample is higher than or the same as the expected TrkB-T':TrkB-FL ratio;
wherein said method further comprises prior to step i): measuring the amount of TrkB-FL and TrkB-ICD in the first sample obtained from the subject, and calculating the TrkB-ICD:TrkB-FL ratio in the first sample and, prior to step ii), measuring the amount of TrkB-FL and TrkB-ICD in the second sample obtained from the subject after administration of an agent to the subject, and calculating the TrkB-ICD:TrkB-FL ratio in the second sample; or measuring the amount of TrkB-FL and TrkB-T' in the first sample obtained from the subject, and calculating the TrkB-T':TrkB-FL ratio in the first sample and, prior to step ii), measuring the amount of TrkB-FL and TrkB-T' in the second sample obtained from the subject after administration of an agent to the subject, and calculating the TrkB-T':TrkB-FL ratio in the second sample.

12. A method for monitoring the efficacy of an AD treatment with an agent, comprising:
i) measuring the amount of TrkB-FL and TrkB-ICD in a first sample obtained from a subject with AD, and
ii) calculating the TrkB-ICD:TrkB-FL ratio in said first sample, and
iii) administering an agent to said subject, and
iv) measuring the amount of TrkB-FL and TrkB-ICD in a second sample obtained from said subject, and
v) calculating the TrkB-ICD:TrkB-FL ratio in said second sample, and
vi) a) determining that the treatment is efficacious if the TrkB-ICD:TrkB-FL ratio in the second sample is lower than the TrkB-ICD:TrkB-FL ratio in the first sample; or b) determining that the treatment is not efficacious if the TrkB-ICD:TrkB-FL ratio in the second sample is higher than the TrkB-ICD:TrkB-FL ratio in the first sample.

13. A method for monitoring the efficacy of an AD treatment with an agent, comprising:
i) measuring the amount of TrkB-FL and TrkB-T' in a first sample obtained from a subject with AD, and
ii) calculating the TrkB-T':TrkB-FL ratio in said first sample, and
iii) administering an agent to said subject, and
iv) measuring the amount of TrkB-FL and TrkB-T' in a second sample obtained from said subject, and
v) calculating the TrkB-T':TrkB-FL ratio in said second sample, and
vi) a) determining that the treatment is efficacious if the TrkB-T':TrkB-FL ratio in the second sample is lower than the TrkB-T':TrkB-FL ratio in the first sample; or b) determining that the treatment is not efficacious if the TrkB-T':TrkB-FL ratio in the second sample is higher than the TrkB-T':TrkB-FL ratio in the first sample.

14. The method of any one of claims 2 to 13, wherein said sample is cerebrospinal fluid (CSF), blood, plasma, serum, or saliva.
